(19) Europäisches Patentamt
European Patent Office
Office européen des brevets

(11) **EP 3 313 193 B1**

(12) **EUROPEAN PATENT SPECIFICATION**

(45) Date of publication and mention
of the grant of the patent:
**24.03.2021 Bulletin 2021/12**

(21) Application number: **16734240.1**

(22) Date of filing: **24.06.2016**

(51) Int Cl.:
*A23F 5/24* *(2006.01)*    *C12N 9/24* *(2006.01)*

(86) International application number:
**PCT/EP2016/064727**

(87) International publication number:
**WO 2016/207384 (29.12.2016 Gazette 2016/52)**

(54) **METHOD FOR PRODUCING A COFFEE EXTRACT**

VERFAHREN ZUR HERSTELLUNG EINES KAFFEEEXTRAKTS

PROCÉDÉ DE PRODUCTION D'UN EXTRAIT DE CAFÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2015 EP 15174117
26.06.2015 EP 15174110**

(43) Date of publication of application:
**02.05.2018 Bulletin 2018/18**

(73) Proprietor: **Novozymes A/S
2880 Bagsvaerd (DK)**

(72) Inventors:
• **EKLÖF, Jens, Magnus
2880 Bagsvaerd (DK)**

• **RASMUSSEN, Louise
2880 Bagsvaerd (DK)**
• **LYNGLEV, Gitte, Budolfsen
2880 Bagsvaerd (DK)**
• **SPODSBERG, Nikolaj
2880 Bagsvaerd (DK)**
• **KROGH, Kristian, Bertel, Roemer, M
2880 Bagsvaerd (DK)**

(74) Representative: **NZ EPO Representatives
Krogshoejvej 36
2880 Bagsvaerd (DK)**

(56) References cited:
**EP-B1- 2 052 078**    **WO-A1-2007/011531**
**CN-A- 103 525 792**    **US-A- 5 714 183**
**US-A1- 2009 325 240**

EP 3 313 193 B1

**Description**

**Reference to a Sequence Listing**

This application contains a Sequence Listing in computer readable form.

**Background of the Invention**

**Field of the Invention**

The present invention relates to enzyme-assisted production of coffee extracts.

**Description of the Related Art**

Coffee extract, i.e., an aqueous solution of soluble solids extracted from the coffee bean, has various industrial applications. It is used, e.g., in the manufacture of instant coffee; in ready-to-drink coffee products such as canned coffee and bottled coffee drinks; and in non-beverage applications such as instant desserts, confectionary products and flavours.
Commercial coffee extracts are typically produced by stagewise thermal processing, a combination of wetting, extraction and hydrolysis stages, which solubilizes a high percentage of the roast and ground coffee solids. Very high temperatures are required to effect thermal hydrolysis and this may lead to off-flavours and to cost and capital intensive processes.
Use of various different enzymes in the production of coffee extracts to improve product quality and process economics has been suggested (see, e.g., US4,983,408, WO2007/011531, US5,714,183). Use of mannanase in the production of a soluble coffee extract has been disclosed in, e.g., WO2007/011531 and US5,714,183.
EP 2 052 078 B1 has disclosed mannanases from *Trichoderma* with improved thermostability after genetic engineering. US 2009/325240 A1 has disclosed thermostable cell wall degrading enzymes including mannanases. CN 103 525 792 A has also disclosed a thermostable mannanase useful in food processing.
It is an object of the present invention to obtain coffee extracts having a high yield of soluble solids.

**Summary of the Invention**

The present inventors have identified novel mannanase enzymes and shown that these are useful for extraction of roast and ground coffee thus giving a high yield of dry matter in the coffee extract obtained.
The present invention therefore relates to method for producing a coffee extract, comprising the steps:

    a. providing roast and ground coffee beans;
    b. optionally performing one or more first extractions of said coffee beans;
    c. adding to said coffee beans, which have optionally been subjected to one or more first extractions, water and an enzyme having mannanase activity;
    d. incubating to make an aqueous coffee extract; and
    e. separating the coffee extract from the extracted coffee beans,

wherein the enzyme having mannanase activity has at least 70% sequence identity to SEQ ID NO: 3.
The inventors have further found out that thermostable mannanase enzymes are particularly useful for extraction of roast and ground coffee. The coffee extracts obtained have a high yield of dry matter. Use of a thermostable mannanase enzyme is an advantage in the production of coffee extracts since this will allow for extraction at higher temperature. In general, extraction at high temperature will give a higher yield. Also, high temperature will reduce microbial growth. Further, in the stagewise extraction process used in commercial production of coffee extracts, an extraction at very high temperature may take place immediately before an extraction wherein a mannanase enzyme is applied, and use of a thermostable mannanase will allow for less cooling between those two extractions.
Preferably, the enzyme having mannanase activity has a melting temperature (Tm) determined by Differential Scanning Calorimetry (DSC) of at least 80°C, preferably at least 85°C or at least 90°C.
Preferably, the incubation in step d. is performed at a temperature of at least 60°C such as at least 65°C, preferably at least 70°C such as at least 75°C or at least 80°C.

**Definitions**

**Mannanase:** In the context of the present invention a "mannanase" is a beta-mannanase. It may be an enzyme defined according to the art as an endo-beta-1,4-mannanase (EC 3.2.1.78) which catalyses the hydrolysis of 1,4-beta-D-mannosidic linkages in mannans, galactomannans and glucomannans, which enzyme has the alternative names mannan endo-1,4-beta-mannosidase; 1,4-beta-D-mannan mannanohydrolase; endo-1,4-beta-mannanase; beta-mannanase B; beta-1,4-mannan 4-mannanohydrolase; endo-beta-mannanase; and beta-D-mannanase. For purposes of the present invention, mannanase activity may be determined using the activity assay described by Staalbrand et al. (1993), Purification and characterization of two beta-mannanases from Trichoderma reesei, J. Biotechnol., 29:229-42. In one aspect, a mannanase to be used in a method of the present invention has at least 20%, e.g., at least 40%, at least 50%, at least 60%, at least 70%, at least 80%, at least 90%, at least 95%, or at least 100% of the mannanase activity of the polypeptide of GENESEQP accession number AXU66990 shown herein as SEQ ID NO: 16.

**Allelic variant:** The term "allelic variant" means any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene.

**Catalytic domain:** The term "catalytic domain" means the region of an enzyme containing the catalytic machinery of the enzyme.

**cDNA:** The term "cDNA" means a DNA molecule that can be prepared by reverse transcription from a mature, spliced, mRNA molecule obtained from a eukaryotic or prokaryotic cell. cDNA lacks intron sequences that may be present in the corresponding genomic DNA. The initial, primary RNA transcript is a precursor to mRNA that is processed through a series of steps, including splicing, before appearing as mature spliced mRNA.

**Coding sequence:** The term "coding sequence" means a polynucleotide, which directly specifies the amino acid sequence of a polypeptide. The boundaries of the coding sequence are generally determined by an open reading frame, which begins with a start codon such as ATG, GTG, or TTG and ends with a stop codon such as TAA, TAG, or TGA. The coding sequence may be a genomic DNA, cDNA, synthetic DNA, or a combination thereof.

**Control sequences:** The term "control sequences" means nucleic acid sequences necessary for expression of a polynucleotide encoding a mature polypeptide of the present invention. Each control sequence may be native (*i.e.,* from the same gene) or foreign (*i.e.,* from a different gene) to the polynucleotide encoding the polypeptide or native or foreign to each other. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide encoding a polypeptide.

**Expression:** The term "expression" includes any step involved in the production of a polypeptide including, but not limited to, transcription, post-transcriptional modification, translation, post-translational modification, and secretion.

**Expression vector:** The term "expression vector" means a linear or circular DNA molecule that comprises a polynucleotide encoding a polypeptide and is operably linked to control sequences that provide for its expression.

**Fragment:** The term "fragment" means a polypeptide having one or more (e.g., several) amino acids absent from the amino and/or carboxyl terminus of a mature polypeptide or domain; wherein the fragment has endo-beta-1,4-mannanase activity. In one aspect, a fragment of the polypeptide of SEQ ID NO: 3 contains at least 350 amino acid residues, at least 375 amino acid residues, or at least 400 amino acid residues. In one aspect, a fragment of the polypeptide of SEQ ID NO: 8 contains at least 300 amino acid residues, at least 315 amino acid residues, or at least 330 amino acid residues. In one aspect, a fragment of the polypeptide of SEQ ID NO: 13 contains at least 300 amino acid residues, at least 315 amino acid residues, or at least 330 amino acid residues.

**High stringency conditions:** The term "high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 65°C.

**Host cell:** The term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

**Isolated:** The term "isolated" means a substance in a form or environment that does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (*e.g.,* recombinant production in a host cell; multiple copies of a gene encoding the substance; and use of a stronger promoter than the promoter naturally associated with the gene encoding the substance).

**Low stringency conditions:** The term "low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 50°C.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide of the polypeptide of SEQ ID NO: 2 is amino acids 18-431 of SEQ ID NO: 2. In one aspect, the mature polypeptide of the polypeptide of SEQ ID NO: 7 is amino acids 18-367 of SEQ ID NO: 7. In one aspect, the mature polypeptide of the polypeptide of SEQ ID NO: 12 is amino acids 18-361 of SEQ ID NO: 12. It is known in the art that a host cell may produce a mixture of two of more different mature polypeptides (*i.e.,* with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.,* having a different C-terminal and/or N-terminal amino acid) as compared to another host cell expressing the same polynucleotide.

**Mature polypeptide coding sequence:** The term "mature polypeptide coding sequence" means a polynucleotide that encodes a mature polypeptide having endo-beta-1,4-mannanase activity. In one aspect, the mature polypeptide coding sequence of SEQ ID NO: 1 is nucleotides 52 to 1545 of SEQ ID NO: 1 or the cDNA sequence thereof. In one aspect, the mature polypeptide coding sequence of SEQ ID NO: 6 is nucleotides 52 to 1219 of SEQ ID NO: 6 or the cDNA sequence thereof. In one aspect, the mature polypeptide coding sequence of SEQ ID NO: 11 is nucleotides 52 to 1200 of SEQ ID NO: 11 or the cDNA sequence thereof.

**Medium stringency conditions:** The term "medium stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 55°C.

**Medium-high stringency conditions:** The term "medium-high stringency conditions" means for probes of at least 100 nucleotides in length, prehybridization and hybridization at 42°C in 5X SSPE, 0.3% SDS, 200 micrograms/ml sheared and denatured salmon sperm DNA, and 35% formamide, following standard Southern blotting procedures for 12 to 24 hours. The carrier material is finally washed three times each for 15 minutes using 2X SSC, 0.2% SDS at 60°C.

**Nucleic acid construct:** The term "nucleic acid construct" means a nucleic acid molecule, either single- or double-stranded, which is isolated from a naturally occurring gene or is modified to contain segments of nucleic acids in a manner that would not otherwise exist in nature or which is synthetic, which comprises one or more control sequences.

**Operably linked:** The term "operably linked" means a configuration in which a control sequence is placed at an appropriate position relative to the coding sequence of a polynucleotide such that the control sequence directs expression of the coding sequence.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

(Identical Residues x 100)/(Length of Alignment – Total Number of Gaps in Alignment)

For purposes of the present invention, the sequence identity between two deoxyribonucleotide sequences is determined using the Needleman-Wunsch algorithm (Needleman and Wunsch, 1970, *supra*) as implemented in the Needle program of the EMBOSS package (EMBOSS: The European Molecular Biology Open Software Suite, Rice et al., 2000, *supra*), preferably version 5.0.0 or later. The parameters used are gap open penalty of 10, gap extension penalty of 0.5, and the EDNAFULL (EMBOSS version of NCBI NUC4.4) substitution matrix. The output of Needle labeled "longest identity" (obtained using the -nobrief option) is used as the percent identity and is calculated as follows:

(Identical Deoxyribonucleotides x 100)/(Length of Alignment – Total Number of Gaps in Alignment)

**Subsequence:** The term "subsequence" means a polynucleotide having one or more (*e.g.,* several) nucleotides absent from the 5' and/or 3' end of a mature polypeptide coding sequence; wherein the subsequence encodes a fragment having endo-beta-1,4-mannanase activity.

**Variant:** The term "variant" means a polypeptide having endo-beta-1,4-mannanase activity comprising an alteration, *i.e.,* a substitution, insertion, and/or deletion, at one or more (*e.g.,* several) positions. A substitution means replacement of the amino acid occupying a position with a different amino acid; a deletion means removal of the amino acid occupying a position; and an insertion means adding one or more amino acids adjacent to and immediately following the amino acid occupying a position.

**Very high stringency conditions:** The term "very high stringency conditions" means for probes of at least 100 nucleotides in length, prehybridization and hybridization at 42°C in 5X SSPE, 0.3% SDS, 200 micrograms/ml sheared and denatured salmon sperm DNA, and 50% formamide, following standard Southern blotting procedures for 12 to 24 hours. The carrier material is finally washed three times each for 15 minutes using 2X SSC, 0.2% SDS at 70°C.

**Very low stringency conditions:** The term "very low stringency conditions" means for probes of at least 100 nucleotides in length, prehybridization and hybridization at 42°C in 5X SSPE, 0.3% SDS, 200 micrograms/ml sheared and denatured salmon sperm DNA, and 25% formamide, following standard Southern blotting procedures for 12 to 24 hours. The carrier material is finally washed three times each for 15 minutes using 2X SSC, 0.2% SDS at 45°C.

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide of the polypeptide of SEQ ID NO: 2 is amino acids 18-431 of SEQ ID NO: 2. In one aspect, the mature polypeptide of the polypeptide of SEQ ID NO: 7 is amino acids 18-367 of SEQ ID NO: 7. In one aspect, the mature polypeptide of the polypeptide of SEQ ID NO: 12 is amino acids 18-361 of SEQ ID NO: 12. In one aspect, the mature polypeptide of the polypeptide of SEQ ID NO: 17 is amino acids 28-319 of SEQ ID NO: 17 (based on N-terminal sequencing and mass spectrometry (MS) of the full-length protein). It is known in the art that a host cell may produce a mixture of two of more different mature polypeptides (*i.e.,* with a different C-terminal and/or N-terminal amino acid) expressed by the same polynucleotide. It is also known in the art that different host cells process polypeptides differently, and thus, one host cell expressing a polynucleotide may produce a different mature polypeptide (*e.g.,* having a different C-terminal and/or N-terminal amino acid) as compared to another host cell expressing the same polynucleotide.

**Thermostable:** In the context of the present invention, a thermostable enzyme having mannanase activity may have a melting temperature ($T_m$) determined by Differential Scanning Calorimetry (DSC) of at least 80°C, preferably at least 85°C, more preferably at least 90°C. The $T_m$ may be determined as described in the Examples.

**Detailed Description of the Invention**

The present invention invention relates to method for producing a coffee extract, comprising the steps:

    a. providing roast and ground coffee beans;
    b. optionally performing one or more first extractions of said coffee beans;
    c. adding to said coffee beans, which have optionally been subjected to one or more first extractions, water and an enzyme having mannanase activity;
    d. incubating to make an aqueous coffee extract; and
    e. separating the coffee extract from the extracted coffee beans,

wherein the enzyme having mannanase activity has at least 70% sequence identity to SEQ ID NO: 3.

In an embodiment, the enzyme having mannanase activity has a sequence identity to the polypeptide of SEQ ID NO: 3 of at least 75%, at least 80%, at least 85%, at least 90%, at least 91%, at least 92%, at least 93%, at least 94%, at least 95%, at least 96%, at least 97%, at least 98%, at least 99%, or 100%. In an embodiment, the enzyme differs by up to 10 amino acids, *e.g.,* 1, 2, 3, 4, 5, 6, 7, 8, 9, or 10, from the polypeptide of SEQ ID NO: 3. In one embodiment, such enzyme is thermostable.

In one embodiment, the enzyme having mannanase activity preferably comprises or consists of the amino acid sequence of SEQ ID NO: 3 or an allelic variant thereof; or is a fragment thereof having mannanase activity. In another aspect, the enzyme comprises or consists of the amino acid sequence of SEQ ID NO: 3. In one embodiment, such enzyme is thermostable.

In a preferred embodiment, the enzyme having mannanase activity has a melting temperature ($T_m$) determined by Differential Scanning Calorimetry (DSC) of at least 80°C, preferably at least 85°C, more preferably at least 90°C. The melting temperature $T_m$ may be determined as described in the Examples.

In a preferred embodiment, incubation is performed at a temperature of at least 60°C such as at least 65°C, preferably at least 70°C such as at least 75°C or at least 80°C.

In another preferred embodiment, incubation is performed at a temperature typically in the range of about 50°C to about 100°C, preferably about 60°C to about 100°C, more preferably about 70°C to about 100°C, even more preferably about 80°C to about 100°C.

In an embodiment, the enzyme having mannanase activity has been isolated.

In an embodiment, the enzyme having mannanase activity is an endo-beta-1,4-mannanase, preferably a GH5 endo-beta-1,4-mannanase, more preferably a GH5_7 endo-beta-1,4-mannanase or a GH5_8 endo-beta-1,4-mannanase. In a preferred embodiment, the enzyme having mannanase activity is a GH5_7 endo-beta-1,4-mannanase. In another preferred embodiment, the enzyme having mannanase activity is a GH5_8 endo-beta-1,4-mannanase.

An enzyme having mannanase activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The enzyme may be a fungal enzyme. For example, the enzyme may be obtained from yeast such as from *Candida, Kluyveromyces, Pichia, Saccharomyces, Schizosaccharomyces,* or *Yarrowia;* or from a filamentous fungus such as from *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria.*

In another embodiment, the enzyme is obtained from *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis.*

In another embodiment, the enzyme is obtained from *Acremonium cellulolyticus, Aspergillus aculeatus, Aspergillus awamori, Aspergillus foetidus, Aspergillus fumigatus, Aspergillus japonicus, Aspergillus nidulans, Aspergillus niger, Aspergillus oryzae, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Fusarium bactridioides, Fusarium cerealis, Fusarium crookwellense, Fusarium culmorum, Fusarium*

*graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia setosa, Thielavia spededonium, Thielavia subthermophila, Thielavia terrestris, Trichoderma harzianum, Trichoderma koningii, Trichoderma longibrachiatum, Trichoderma reesei,* or *Trichoderma viride.*

In one embodiment, the enzyme is obtained from *Talaromyces, e.g.,* from *Talaromyces leycettanus.*

In another embdodiment, the enzyme is obtained from *Chaetomium, e.g.,* from *Chaetomium virescens.*

In another embodiment, the enzyme is obtained from *Sordaria, e.g.,* from *Sordaria macrospora.*

In another embodiment, the enzyme is obtained from *Caldicellulosiruptor, e.g.,* from *Caldicellulosiruptor saccharolyticus.*

It will be understood that for the aforementioned species, the invention encompasses both the perfect and imperfect states, and other taxonomic equivalents, e.g., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (*e.g.,* soil, composts, water, etc.) or DNA samples obtained directly from natural materials (*e.g.,* soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.,* Sambrook *et al.,* 1989, *supra*).

In one embodiment, the enzyme having mannanase activity is not obtained from *Aspergillus niger.*

The method of the present invention can be applied to fresh roast and ground coffee beans or to roasted coffee grounds which have been previously extracted with water.

In a preferred embodiment, the roast and ground coffee beans have been partially extracted.

In an embodiment, one first extraction is performed in step b.

The method of the invention can be applied to ground coffee beans obtained by conventional soluble coffee processing. Therein, roast coffee is typically ground and (thermally) extracted with water in multiple stages. A two-stage execution is typical in the art, wherein the first stage comprises wetting the coffee grounds, recovery of flavour and extraction of the readily soluble components (such as caffeine, minerals and simple sugars). The second stage is typically a hydrolysis stage, where large coffee bio-polymers and bound components are broken down to smaller water-soluble ones. In the first stage, the roast coffee is typically extracted with water at or below 100°C. The grounds from this extraction, which may be referred to as "atmospheric grounds", are then extracted with superheated water at temperatures between 140°C and 180°C or even higher. The partially extracted grounds from the superheated extraction may be referred to as "super-heated grounds".

If the method of the invention is applied to partially extracted grounds, a first extraction may be carried out by adding the roast and ground coffee which may have an average particle size of about 900 micron to a jacketed stirred tank which contains water, wherein the solids to water ratio is about 1:5. The slurry is stirred, heated indirectly to a temperature of less than about 140°C, preferably in the range of about 85-90°C, and held at this temperature for about 30 minutes. The slurry is then discharged from the vessel and the subsequent grounds and extract separated using a filter. The partially extracted grounds are subjected to a second extraction according to the invention and the extract produced in the first extraction (step b) may be blended with the second extract obtained in step e.

Also, a multi-stage execution (i.e., more than two extractions) is typical in the art. After the first stage, multiple subsequent stages are performed. The method of the invention may be part of such multi-stage extraction. Partially extracted grounds which have been subjected to one or more first extractions are subjected to an extraction according to the invention and the extract produced in the one or more previous extractions may be blended with the extract obtained in step e.

In the context of the present invention, partially extracted ground coffee beans or partially extracted coffee grounds means that the ground coffee beans have been subjected to at least one extraction. Such partially extracted ground coffee beans may also be referred to as spent coffee grounds.

The method of the invention may, in general, be applied to roast and ground coffee comprising roasted beans which were ground to an average particle size of between about 0.1 to about 5 mm, preferably between about 0.2 to about 1 mm.

In addition, a flavour management pre-treatment step can be added to the method of the invention to recover the aroma compounds or aromatic constituents of the coffee prior to the extraction and/or hydrolysis stages. Useful processes include, but are not limited to, those de-scribed in EP 0 489 401. A practical execution includes wetting roast and ground coffee with water in a vessel in a ratio of about 1:0.5 by weight. Vacuum is applied to the vessel (e.g., about 150 mbar) and then low pressure steam is applied to the bed of wetted grounds for up to about 4 to 8 minutes to evaporate aroma compounds from the roast and ground coffee. Volatile compounds drawn off are condensed, for example at about 5°C and retained to be added back to extracts or extracted solids.

The method of the invention can be practiced on roast coffee which has been steamed-purged at low pressure to extract volatile flavour components, as described above.

The method of the invention may be applied to any type of coffee grounds with hydrolysable matter known to those skilled in the art, such as de-oiled coffee grounds, decaffeinated coffee grounds, wet-milled coffee grounds, asparaginase-treated coffee grounds, etc.

The enzymatic treatment of the roast and ground coffee beans is to be performed at a temperature where the enzymes are active and for sufficiently long time to permit enzyme reaction.

In one possible batch mode of operation, after the enzymatic reaction is essentially completed, the mixture is subjected to a gross separation, for example centrifugation or belt filtration, which removes most of the insoluble solids. The separated extract, still containing fine particulates, oil and enzyme protein, is recirculated through a cross-flow membrane device, which removes all insolubles and can also remove enzyme. Most or all of the enzyme remains in the membrane retentate and may be recycled to the reaction.

In one possible mode of operation, semi-permeable membrane permeate is constantly withdrawn during the enzyme reaction, i.e. a portion of the reaction mixture is continuously cir-culated through the cross-flow semi-permeable membrane separation cell. The process can be operated in a semi-continuous mode, wherein permeate is withdrawn until the volume in the re-action vessel diminishes to the point where its viscosity or the pressure drop becomes high. At this point, some retentate is purged and fresh coffee slurry fed and some fresh enzyme added. The purged retentate can be discarded or can be washed to recover the enzyme which is then re-used. The enzyme in the remaining (non-purged) retentate is retained and re-used.

Alternatively, fresh feed slurry may be continuously added to the feed tank together with some enzyme with a purge drawn from the recycle stream of equal volume.

In any event, running the process in a semi-continuous or continuous mode of operation permits permeation of solubilized components out of the reaction zone before they can be fur-ther broken down.

If the method of the invention is used for treating grounds from roast and ground coffee which has been previously extracted with water and/or thermally hydrolysed, the extract obtained from the method of this invention can be combined with the extracts obtained beforehand.

Where atmospheric grounds are used as the feed to the method of the invention, the extract produced may be combined with the extract obtained during the atmospheric extraction stage. The extracts are combined based on the ratio of extracted roasted yields from each stage. The combined extract is then concentrated, aromatised and dried as is conventional in the art.

The coffee extract can be dehydrated, such as a soluble coffee or dry mix composition, or it can be a ready-to-drink coffee product, a liquid mix composition, a frozen composition or a liquid concentrate composition. The coffee extract of the invention can also be used in non-beverage applications, such as instant desserts or confectionery products etc.

The processes to make those coffee compositions from soluble coffee extracts are known to a person skilled in the art.

In the method of the invention, water and enzyme is added to the coffee beans which may have been subjected to one or more first extractions.

Water may, e.g., be added so that the final concentration of dry matter is between 2%-30% (w/w), preferably between 5%-20% (w/w), such as about 10% (w/w).

The enzyme having mannanase activity may be added at a concentration of at least 0.001 g enzyme protein /kg dry matter, preferably at least 0.005 g enzyme protein /kg dry matter, such as at a concentration of 0.001-1 g enzyme protein /kg dry matter, preferably 0.005-0.5 g enzyme protein /kg dry matter.

The enzyme having mannanase activity may be added at a concentration of at least 0.001 g enzyme protein /kg coffee beans, preferably at least 0.005 g enzyme protein /kg coffee beans, such as at a concentration of 0.001-0.5 g enzyme protein /kg coffee beans, preferably 0.005-0.2 g enzyme protein /kg coffee beans.

The enzyme having mannanase activity is preferably added as an enzymatic preparation characterized in that at least 5%, preferably at least 10% or at least 20%, of the total protein in the preparation is an enzyme having mannanase activity as its predominant enzymatic activity.

The enzyme having mannanase activity may be added as a mixture with other enzymes such as, e.g., cellulase and/or galactanase enzyme(s).

In the method of the invention, after the water and the enzyme has been added to the roast and ground coffee beans which have optionally been subjected to one or more first extractions, the composition comprising the coffee beans, the water and the enzyme is incubated to make an aqueous coffee extract.

The incubation is to be performed at a temperature where the enzyme is active, typically in the range of about 25°C to about 100°C. In the aspects of the invention where a thermostable enzyme is used, incubation may be performed at a temperature typically in the range of about 50°C to about 100°C, preferably about 60°C to about 100°C, more preferably about 70°C to about 100°C, even more preferably about 80°C to about 100°C.

The incubation may be performed for about 1 to about 48 hours, preferably about 2 to about 24 hours or about 4 to about 24 hours to permit enzyme reaction.

After the incubation, the coffee extract is separated from the extracted coffee beans by any means known in the art.

In one embodiment, the roast and ground coffee beans are subjected to one or more first extractions before step c, said one or more first extractions being denoted as step b, and a steam explosion is performed after step b and before step c. Alternatively, if more than one first extractions are performed, the steam explosion may be performed in between some of the first extractions and before step c.

In one embodiment, the roast and ground coffee beans are subjected to one or more first extractions before step c, said one or more first extractions being denoted as step b, and a second milling of the coffee beans is performed after step b and before step c. Alternatively, if more than one first extractions are performed, the second milling may be performed in between some of the first extractions and before step c.

In one embodiment, the roast and ground coffee beans are subjected to one or more first extractions before step c, said first extraction(s) being denoted as step b, and at least 8% by weight, preferably at least 10% by weight, of the dry matter of the partially extracted coffee beans obtained after step b is recovered in the coffee extract obtained in step e. In another embodiment, the roast and ground coffee beans are subjected to one or more first extractions before step c, said first extraction(s) being denoted as step b, and at least 12% by weight, preferably at least 14% by weight, of the dry matter of the partially extracted coffee beans obtained after step b is recovered in the coffee extract obtained in step e. In another embodiment, the roast and ground coffee beans are subjected to one or more first extractions before step c, said first extraction(s) being denoted as step b, and 8-40% by weight, preferably 10-30% or 12-25% by weight, of the dry matter of the partially extracted coffee beans obtained after step b is recovered in the coffee extract obtained in step e.

In one embodiment, the roast and ground coffee beans are subjected to one or more first extractions before step c and the coffee extract obtained in step e comprises at least 100% more dry matter, preferably at least 200% or at least 300% more dry matter, than a coffee extract prepared by a similar method without the addition of an enzyme having mannanase activity.

In some applications, the content of free monosaccharides in the coffee extract is important, since these may influence the taste of the coffee extract.

In one embodiment, the roast and ground coffee beans are subjected to one or more first extractions before step c and the coffee extract obtained in step e comprises at least 2% by weight, e.g. at least 5% or at least 8% by weight, of free monosaccharides based on the weight of the total sugars as monosaccharides. In another embodiment, the roast and ground coffee beans are subjected to one or more first extractions before step c and the coffee extract obtained in step e comprises 2-30% by weight, e.g. 5-30% or 8-30% by weight, of free monosaccharides based on the weight of the total sugars as monosaccharides.

In one embodiment, the roast and ground coffee beans are subjected to one or more first extractions before step c and the coffee extract obtained in step e comprises at least 2% by weight of free mannose based on the total weight of soluble coffee solids. In another embodiment, the roast and ground coffee beans are subjected to one or more first extractions before step c and the coffee extract obtained in step e comprises 2-5% by weight of free mannose based on the total weight of soluble coffee solids.

In one embodiment, the roast and ground coffee beans are subjected to one or more first extractions before step c and the free mannose content in the coffee extract obtained in step e is at least 5% by weight, preferably at least 10% by weight, of the total mannose content in said coffee extract. In another embodiment, the roast and ground coffee beans are subjected to one or more first extractions before step c and the free mannose content in the coffee extract obtained in step e is 5-30% by weight, preferably 10-25% by weight, of the total mannose content in said coffee extract.

Total mannose in the coffee extract in the context of the present invention means solubilized free mannose plus mannose bound in solubilized oligosaccharides.

Little or no content of glucose in coffee extracts is a quality parameter, and the glucose content has to be below 2.46 % by weight to comply with the Commercial Item Description (CID) of May 16, 2013, authorized by the U.S. Department of Agriculture (USDA) (http://www.ams.usda.gov/AMSv1.0/getfile?dDocName=STELPRD3237484).

In one embodiment, the coffee extract obtained in step e comprises below 2.46% by weight, preferably below 2% by weight, more preferably below 1% or below 0.5% by weight, of total glucose based on the total weight of soluble coffee solids.

In one embodiment, the coffee extract obtained in step e comprises at least 15% by weight of total mannose based on the total weight of soluble coffee solids. In another embodiment, the coffee extract comprises 15-30% by weight of total mannose based on the total weight of soluble coffee solids.

### Polypeptides Having Endo-beta-1,4-mannanase Activity

In another aspect, the present disclosure relates to polypeptides having endo-beta-1,4-mannanase activity and polynucleotides encoding the polypeptides. The invention also relates to nucleic acid constructs, vectors, and host cells comprising the polynucleotides as well as methods of producing and using the polypeptides.

In an embodiment, the present disclosure relates to polypeptides having a sequence identity to the polypeptide of SEQ ID NO: 3 of at least 75%, e.g., at least 80%, at least 85%, at least 90%, at least 91%, at least 92%, at least 93%, at least 94%, at least 95%, at least 96%, at least 97%, at least 98%, at least 99%, or 100%, which have endo-beta-1,4-mannanase activity. In one aspect, the polypeptides differ by up to 10 amino acids, e.g., 1, 2, 3, 4, 5, 6, 7, 8, 9, or 10, from the polypeptide of SEQ ID NO: 3.

In an embodiment, the present disclosure relates to polypeptides having a sequence identity to the polypeptide of SEQ ID NO: 8 of at least 90%, e.g., at least 91%, at least 92%, at least 93%, at least 94%, at least 95%, at least 96%, at least 97%, at least 98%, at least 99%, or 100%, which have endo-beta-1,4-mannanase activity. In one aspect, the polypeptides differ by up to 10 amino acids, e.g., 1, 2, 3, 4, 5, 6, 7, 8, 9, or 10, from the polypeptide of SEQ ID NO: 8.

In an embodiment, the present disclosure relates to polypeptides having a sequence identity to the polypeptide of SEQ ID NO: 13 of at least 80%, e.g., at least 85%, at least 90%, at least 91%, at least 92%, at least 93%, at least 94%, at least 95%, at least 96%, at least 97%, at least 98%, at least 99%, or 100%, which have endo-beta-1,4-mannanase activity. In one aspect, the polypeptides differ by up to 10 amino acids, e.g., 1, 2, 3, 4, 5, 6, 7, 8, 9, or 10, from the polypeptide of SEQ ID NO: 13.

In an embodiment, the polypeptide has been isolated.

In one embodiment, a polypeptide of the present disclosure preferably comprises or consists of the amino acid sequence of SEQ ID NO: 3 or an allelic variant thereof; or is a fragment thereof having endo-beta-1,4-mannanase activity. In another aspect, the polypeptide comprises or consists of the amino acid sequence of SEQ ID NO: 3.

In one embodiment, a polypeptide of the present disclosure preferably comprises or consists of the amino acid sequence of SEQ ID NO: 8 or an allelic variant thereof; or is a fragment thereof having endo-beta-1,4-mannanase activity. In another aspect, the polypeptide comprises or consists of the amino acid sequence of SEQ ID NO: 8.

In one embodiment, a polypeptide of the present disclosure preferably comprises or consists of the amino acid sequence of SEQ ID NO: 13 or an allelic variant thereof; or is a fragment thereof having endo-beta-1,4-mannanase activity. In another aspect, the polypeptide comprises or consists of the amino acid sequence of SEQ ID NO: 13.

In another embodiment, the present disclosure relates to a polypeptide having endo-beta-1,4-mannanase activity encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 1, (ii) the cDNA sequence thereof], or (iii) the full-length complement of (i) or (ii) (Sambrook et al., 1989, Molecular Cloning, A Laboratory Manual, 2d edition, Cold Spring Harbor, New York).

In another embodiment, the present disclosure relates to a polypeptide having endo-beta-1,4-mannanase activity encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 6, (ii) the cDNA sequence thereof], or (iii) the full-length complement of (i) or (ii).

In another embodiment, the present disclosure relates to a polypeptide having endo-beta-1,4-mannanase activity encoded by a polynucleotide that hybridizes under very low stringency conditions, low stringency conditions, medium stringency conditions, medium-high stringency conditions, high stringency conditions, or very high stringency conditions with (i) the mature polypeptide coding sequence of SEQ ID NO: 11, (ii) the cDNA sequence thereof], or (iii) the full-length complement of (i) or (ii).

The polynucleotide of any of SEQ ID NO: 1, 6 or 11 or a subsequence of any of these, as well as the polypeptide of SEQ ID NO: 2, 7 or 12 or a fragment of any of these, may be used to design nucleic acid probes to identify and clone DNA encoding polypeptides having endo-beta-1,4-mannanase activity from strains of different genera or species according to methods well known in the art. In particular, such probes can be used for hybridization with the genomic DNA or cDNA of a cell of interest, following standard Southern blotting procedures, in order to identify and isolate the corresponding gene therein. Such probes can be considerably shorter than the entire sequence, but should be at least 15, *e.g.,* at least 25, at least 35, or at least 70 nucleotides in length. Preferably, the nucleic acid probe is at least 100 nucleotides in length, *e.g.,* at least 200 nucleotides, at least 300 nucleotides, at least 400 nucleotides, at least 500 nucleotides, at least 600 nucleotides, at least 700 nucleotides, at least 800 nucleotides, or at least 900 nucleotides in length. Both DNA and RNA probes can be used. The probes are typically labeled for detecting the corresponding gene (for example, with $^{32}P$, $^{3}H$, $^{35}S$, biotin, or avidin). Such probes are encompassed by the present invention.

A genomic DNA or cDNA library prepared from such other strains may be screened for DNA that hybridizes with the probes described above and encodes a polypeptide having endo-beta-1,4-mannanase activity. Genomic or other DNA from such other strains may be separated by agarose or polyacrylamide gel electrophoresis, or other separation techniques. DNA from the libraries or the separated DNA may be transferred to and immobilized on nitrocellulose or other suitable carrier material. In order to identify a clone or DNA that hybridizes with SEQ ID NO: 1 or a subsequence thereof, the carrier material is used in a Southern blot.

For purposes of the present invention, hybridization indicates that the polynucleotide hybridizes to a labeled nucleic acid probe corresponding to (i) SEQ ID NO: 1, 6 or 11; (ii) the mature polypeptide coding sequence of SEQ ID NO: 1, 6 or 11; (iii) the cDNA sequence thereof]; (iv) the full-length complement thereof; or (v) a subsequence thereof; under very low to very high stringency conditions. Molecules to which the nucleic acid probe hybridizes under these conditions can be detected using, for example, X-ray film or any other detection means known in the art.

In another embodiment, the present disclosure relates to a polypeptide having endo-beta-1,4-mannanase activity encoded by a polynucleotide having a sequence identity to the mature polypeptide coding sequence of SEQ ID NO: 1 or the cDNA sequence thereof of at least 75%, e.g., at least 80%, at least 85%, at least 90%, at least 91%, at least 92%, at least 93%, at least 94%, at least 95%, at least 96%, at least 97%, at least 98%, at least 99%, or 100%.

In another embodiment, the present disclosure relates to a polypeptide having endo-beta-1,4-mannanase activity encoded by a polynucleotide having a sequence identity to the mature polypeptide coding sequence of SEQ ID NO: 6 or the cDNA sequence thereof of at least 90%, e.g., at least 91%, at least 92%, at least 93%, at least 94%, at least 95%, at least 96%, at least 97%, at least 98%, at least 99%, or 100%.

In another embodiment, the present disclosure relates to a polypeptide having endo-beta-1,4-mannanase activity encoded by a polynucleotide having a sequence identity to the mature polypeptide coding sequence of SEQ ID NO: 11 or the cDNA sequence thereof of at least 80%, *e.g.,* at least 85%, at least 90%, at least 91%, at least 92%, at least 93%, at least 94%, at least 95%, at least 96%, at least 97%, at least 98%, at least 99%, or 100%.

In another embodiment, the present disclosure relates to variants of the polypeptide of any of SEQ ID NO: 3, 8 or 13 comprising a substitution, deletion, and/or insertion at one or more (*e.g.,* several) positions. In an embodiment, the number of amino acid substitutions, deletions and/or insertions introduced into the polypeptide of any of SEQ ID NO: 3, 8 or 13 is up to 10, *e.g.,* 1, 2, 3, 4, 5, 6, 7, 8, 9, or 10. The amino acid changes may be of a minor nature, that is conservative amino acid substitutions or insertions that do not significantly affect the folding and/or activity of the protein; small deletions, typically of 1-30 amino acids; small amino- or carboxyl-terminal extensions, such as an amino-terminal methionine residue; a small linker peptide of up to 20-25 residues; or a small extension that facilitates purification by changing net charge or another function, such as a poly-histidine tract, an antigenic epitope or a binding domain.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Alternatively, the amino acid changes are of such a nature that the physico-chemical properties of the polypeptides are altered. For example, amino acid changes may improve the thermal stability of the polypeptide, alter the substrate specificity, change the pH optimum, and the like.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for endo-beta-1,4-mannanase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64. The identity of essential amino acids can also be inferred from an alignment with a related polypeptide.

Single or multiple amino acid substitutions, deletions, and/or insertions can be made and tested using known methods of mutagenesis, recombination, and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; U.S. Patent No. 5,223,409; WO 92/06204), and region-directed mutagenesis (Derbyshire et al., 1986, Gene 46: 145; Ner et al., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

The polypeptide may be a hybrid polypeptide in which a region of one polypeptide is fused at the N-terminus or the C-terminus of a region of another polypeptide.

The polypeptide may be a fusion polypeptide or cleavable fusion polypeptide in which another polypeptide is fused at the N-terminus or the C-terminus of the polypeptide of the present invention. A fusion polypeptide is produced by fusing a polynucleotide encoding another polypeptide to a polynucleotide of the present invention. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also be constructed using intein technology in which fusion polypeptides are created post-translationally (Cooper et al., 1993, EMBO J. 12: 2575-2583; Dawson et al., 1994, Science 266: 776-779).

A fusion polypeptide can further comprise a cleavage site between the two polypeptides. Upon secretion of the fusion protein, the site is cleaved releasing the two polypeptides. Examples of cleavage sites include, but are not limited to, the sites disclosed in Martin et al., 2003, J. Ind. Microbiol. Biotechnol. 3: 568-576; Svetina et al., 2000, J. Biotechnol. 76: 245-251; Rasmussen-Wilson et al., 1997, Appl. Environ. Microbiol. 63: 3488-3493; Ward et al., 1995, Biotechnology 13: 498-503; and Contreras et al., 1991, Biotechnology 9: 378-381; Eaton et al., 1986, Biochemistry 25: 505-512; Collins-Racie et al., 1995, Biotechnology 13: 982-987; Carter et al., 1989, Proteins: Structure, Function, and Genetics 6: 240-248; and Stevens, 2003, Drug Discovery World 4: 35-48.

## Sources of Polypeptides Having Endo-beta-1,4-mannanase Activity

A polypeptide having endo-beta-1,4-mannanase activity of the present invention may be obtained from microorganisms of any genus. For purposes of the present invention, the term "obtained from" as used herein in connection with a given source shall mean that the polypeptide encoded by a polynucleotide is produced by the source or by a strain in which the polynucleotide from the source has been inserted. In one aspect, the polypeptide obtained from a given source is secreted extracellularly.

The polypeptide may be a fungal polypeptide. For example, the polypeptide may be a yeast polypeptide such as a *Candida, Kluyveromyces, Pichia, Saccharomyces, Schizosaccharomyces,* or *Yarrowia* polypeptide; or a filamentous fungal polypeptide such as an *Acremonium, Agaricus, Alternaria, Aspergillus, Aureobasidium, Botryospaeria, Ceriporiopsis, Chaetomidium, Chrysosporium, Claviceps, Cochliobolus, Coprinopsis, Coptotermes, Corynascus, Cryphonectria, Cryptococcus, Diplodia, Exidia, Filibasidium, Fusarium, Gibberella, Holomastigotoides, Humicola, Irpex, Lentinula, Leptospaeria, Magnaporthe, Melanocarpus, Meripilus, Mucor, Myceliophthora, Neocallimastix, Neurospora, Paecilomyces, Penicillium, Phanerochaete, Piromyces, Poitrasia, Pseudoplectania, Pseudotrichonympha, Rhizomucor, Schizophyllum, Scytalidium, Talaromyces, Thermoascus, Thielavia, Tolypocladium, Trichoderma, Trichophaea, Verticillium, Volvariella,* or *Xylaria* polypeptide.

In another aspect, the polypeptide is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* polypeptide.

In another aspect, the polypeptide is an *Acremonium cellulolyticus, Aspergillus aculeatus, Aspergillus awamori, Aspergillus foetidus, Aspergillus fumigatus, Aspergillus japonicus, Aspergillus nidulans, Aspergillus niger, Aspergillus oryzae, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola grisea, Humicola insolens, Humicola lanuginosa, Irpex lacteus, Mucor miehei, Myceliophthora thermophila, Neurospora crassa, Penicillium funiculosum, Penicillium purpurogenum, Phanerochaete chrysosporium, Thielavia achromatica, Thielavia albomyces, Thielavia albopilosa, Thielavia australeinsis, Thielavia fimeti, Thielavia microspora, Thielavia ovispora, Thielavia peruviana, Thielavia setosa, Thielavia spededonium, Thielavia subthermophila, Thielavia terrestris, Trichoderma harzianum, Trichoderma koningii, Trichoderma longibrachiatum, Trichoderma reesei,* or *Trichoderma viride* polypeptide.

In one aspect, the polypeptide is a *Talaromyces* polypeptide, *e.g.,* a polypeptide obtained from *Talaromyces*

*leycettanus.*

In another aspect, the polypeptide is a *Chaetomium* polypeptide, *e.g.,* a polypeptide obtained from *Chaetomium virescens.*

In another aspect, the polypeptide is a *Sordaria* polypeptide, *e.g.,* a polypeptide obtained from *Sordaria macrospora.*

It will be understood that for the aforementioned species, the disclosure encompasses both the perfect and imperfect states, and other taxonomic equivalents, *e.g.,* anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSMZ), Centraalbureau Voor Schimmelcultures (CBS), and Agricultural Research Service Patent Culture Collection, Northern Regional Research Center (NRRL).

The polypeptide may be identified and obtained from other sources including microorganisms isolated from nature (*e.g.,* soil, composts, water, etc.) or DNA samples obtained directly from natural materials (*e.g.,* soil, composts, water, etc.) using the above-mentioned probes. Techniques for isolating microorganisms and DNA directly from natural habitats are well known in the art. A polynucleotide encoding the polypeptide may then be obtained by similarly screening a genomic DNA or cDNA library of another microorganism or mixed DNA sample. Once a polynucleotide encoding a polypeptide has been detected with the probe(s), the polynucleotide can be isolated or cloned by utilizing techniques that are known to those of ordinary skill in the art (see, *e.g.,* Sambrook *et al.,* 1989, *supra).*

**Catalytic Domains**

In one embodiment, the present disclosure also relates to catalytic domains having a sequence identity to amino acids 75 to 414 of SEQ ID NO: 3 of at least 75%, *e.g.,* at least 80%, at least 85%, at least 90%, at least 95%, at least 96%, at least 97%, at least 98%, at least 99%, or 100%. In one aspect, the catalytic domains comprise amino acid sequences that differ by up to 10 amino acids, *e.g.,* 1, 2, 3, 4, 5, 6, 7, 8, 9, or 10, from amino acids 75 to 414 of SEQ ID NO: 3.

The catalytic domain preferably comprises or consists of amino acids 75 to 414 of SEQ ID NO: 3 or an allelic variant thereof; or is a fragment thereof having endo-beta-1,4-mannanase activity.

In another embodiment, the present disclosure also relates to catalytic domains encoded by polynucleotides that hybridize under very low stringency conditions, low stringency conditions, medium stringency conditions, medium-high stringency conditions, high stringency conditions, or very high stringency conditions (as defined above) with (i) the nucleotides 317 to 1545 of SEQ ID NO: 1, (ii) the cDNA sequence thereof, or (iii) the full-length complement of (i) or (ii) (Sambrook *et al.,* 1989, *supra).*

In another embodiment, the present disclosure also relates to catalytic domains encoded by polynucleotides having a sequence identity to nucleotides 317 to 1545 of SEQ ID NO: 1 or the cDNA sequence thereof of at least 75%, *e.g.,* at least 80%, at least 85%, at least 90%, at least 95%, at least 96%, at least 97%, at least 98%, at least 99%, or 100%.

The polynucleotide encoding the catalytic domain preferably comprises or consists of nucleotides 317 to 1545 of SEQ ID NO: 1.

In another embodiment, the present disclosure also relates to catalytic domain variants of amino acids 75 to 414 of SEQ ID NO: 3 comprising a substitution, deletion, and/or insertion at one or more (*e.g.,* several) positions. In one aspect, the number of amino acid substitutions, deletions and/or insertions introduced into the sequence of amino acids 75 to 414 of SEQ ID NO: 3 is up to 10, *e.g.,* 1, 2, 3, 4, 5, 6, 8, 9, or 10.

**Binding Domains**

In one embodiment, the present disclosure also relates to a CBM1 binding domains having a sequence identity to amino acids 1 to 37 of SEQ ID NO: 3 of at least 75%, *e.g.,* at least 80%, at least 85%, at least 90%, at least 91%, at least 92%, at least 93%, at least 94%, at least 95%, at least 96%, at least 97%, at least 98%, at least 99%, or 100%. In one aspect, the CBM1 binding domains comprise amino acid sequences that differ by up to 10 amino acids, *e.g.,* 1, 2, 3, 4, 5, 6, 7, 8, 9, or 10, from amino acids 1 to 37 of SEQ ID NO: 3.

The CBM1 binding domain preferably comprises or consists of amino acids 1 to 37 of SEQ ID NO: 3 or an allelic variant thereof; or is a fragment thereof having CBM1 binding activity.

In another embodiment, the present disclosure also relates to CBM1 binding domains encoded by polynucleotides that hybridize under very low stringency conditions, low stringency conditions, medium stringency conditions, medium-high stringency conditions, high stringency conditions, or very high stringency conditions (as defined above) with (i) the nucleotides 1 to 111 of SEQ ID NO: 1, (ii) the cDNA sequence thereof, or (iii) the full-length complement of (i) or (ii) (Sambrook *et al.,* 1989, *supra).*

In another embodiment, the present disclosure also relates to CBM1 binding domains encoded by polynucleotides having a sequence identity to nucleotides 1 to 111 of SEQ ID NO: 1 of at least 75%, *e.g.,* at least 80%, at least 85%, at least 90%, at least 91%, at least 92%, at least 93%, at least 94%, at least 95%, at least 96%, at least 97%, at least 98%, at least 99%, or 100%.

The polynucleotide encoding the CBM1 binding domain preferably comprises or consists of nucleotides 1 to 111 of SEQ ID NO: 1.

In another embodiment, the present disclosure also relates to CBM1 binding domain variants of amino acids 1 to 37 of SEQ ID NO: 3 comprising a substitution, deletion, and/or insertion at one or more (*e.g.,* several) positions. In one aspect, the number of amino acid substitutions, deletions and/or insertions introduced into the sequence of amino acids 1 to 37 of SEQ ID NO: 3 is up to 10, *e.g.,* 1, 2, 3, 4, 5, 6, 8, 9, or 10.

A catalytic domain operably linked to the CBM1 binding domain may be from a hydrolase, isomerase, ligase, lyase, oxidoreductase, or transferase, e.g., an aminopeptidase, amylase, carbohydrase, carboxypeptidase, catalase, cellobiohydrolase, cellulase, chitinase, cutinase, cyclodextrin glycosyltransferase, deoxyribonuclease, endoglucanase, esterase, alpha-galactosidase, beta-galactosidase, glucoamylase, alpha-glucosidase, beta-glucosidase, invertase, laccase, lipase, mannosidase, mutanase, oxidase, pectinolytic enzyme, peroxidase, phytase, polyphenoloxidase, proteolytic enzyme, ribonuclease, transglutaminase, xylanase, or beta-xylosidase. The polynucleotide encoding the catalytic domain may be obtained from any prokaryotic, eukaryotic, or other source.

## Polynucleotides

The present disclosure also relates to polynucleotides encoding a polypeptide, a catalytic domain, or CBM1 binding domain of the present invention, as described herein. In an embodiment, the polynucleotide encoding the polypeptide, catalytic domain, or CBM1 binding domain of the present invention has been isolated.

The techniques used to isolate or clone a polynucleotide are known in the art and include isolation from genomic DNA or cDNA, or a combination thereof. The cloning of the polynucleotides from genomic DNA can be effected, *e.g.,* by using the well known polymerase chain reaction (PCR) or antibody screening of expression libraries to detect cloned DNA fragments with shared structural features. See, *e.g.,* Innis et al., 1990, PCR: A Guide to Methods and Application, Academic Press, New York. Other nucleic acid amplification procedures such as ligase chain reaction (LCR), ligation activated transcription (LAT) and polynucleotide-based amplification (NASBA) may be used. The polynucleotides may be cloned from a strain of *Talaromyces, Chaetomium,* or *Sordaria,* or a related organism and thus, for example, may be an allelic or species variant of the polypeptide encoding region of the polynucleotide.

Modification of a polynucleotide encoding a polypeptide of the present invention may be necessary for synthesizing polypeptides substantially similar to the polypeptide. The term "substantially similar" to the polypeptide refers to non-naturally occurring forms of the polypeptide. These polypeptides may differ in some engineered way from the polypeptide isolated from its native source, *e.g.,* variants that differ in specific activity, thermostability, pH optimum, or the like. The variants may be constructed on the basis of the polynucleotide presented as the mature polypeptide coding sequence of SEQ ID NO: 1, 6 or 11, or the cDNA sequence thereof, *e.g.,* a subsequence thereof, and/or by introduction of nucleotide substitutions that do not result in a change in the amino acid sequence of the polypeptide, but which correspond to the codon usage of the host organism intended for production of the enzyme, or by introduction of nucleotide substitutions that may give rise to a different amino acid sequence. For a general description of nucleotide substitution, see, *e.g.,* Ford et al., 1991, Protein Expression and Purification 2: 95-107.

## Nucleic Acid Constructs

The present disclosure also relates to nucleic acid constructs comprising a polynucleotide of the present invention operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the *Bacillus amyloliquefaciens* alpha-amylase gene (*amyQ*), *Bacillus licheniformis* alpha-amylase gene (*amyL*), *Bacillus licheniformis* penicillinase gene (*penP*), *Bacillus stearothermophilus* maltogenic amylase gene (*amyM*), *Bacillus subtilis* levansucrase gene (*sacB*), *Bacillus subtilis xylA* and *xylB* genes, *Bacillus thuringiensis cryIIIA* gene (Agaisse and Lereclus, 1994, Molecular Microbiology 13: 97-107), *E. coli lac* operon, *E. coli trc* promoter (Egon et al., 1988, Gene 69: 301-315), *Streptomyces coelicolor* agarase gene (*dagA*), and prokaryotic beta-lactamase gene (Villa-Kamaroff et al., 1978, Proc. Natl. Acad. Sci. USA 75: 3727-3731), as well as the *tac* promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook *et al.,* 1989, *supra.* Examples of tandem promoters are disclosed in WO 99/43835.

Examples of suitable promoters for directing transcription of the nucleic acid constructs of the present invention in a filamentous fungal host cell are promoters obtained from the genes for *Aspergillus nidulans* acetamidase, *Aspergillus niger* neutral alpha-amylase, *Aspergillus niger* acid stable alpha-amylase, *Aspergillus niger* or *Aspergillus awamori* glucoamylase (*glaA*), *Aspergillus oryzae* TAKA amylase, *Aspergillus oryzae* alkaline protease, *Aspergillus oryzae* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2-tpi promoter (a modified promoter from an *Aspergillus* neutral alpha-amylase gene in which the untranslated leader has been replaced by an untranslated leader from an *Aspergillus* triose phosphate isomerase gene; non-limiting examples include modified promoters from an *Aspergillus niger* neutral alpha-amylase gene in which the untranslated leader has been replaced by an untranslated leader from an *Aspergillus nidulans* or *Aspergillus oryzae* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO-1), *Saccharomyces cerevisiae* galactokinase (GAL1), *Saccharomyces cerevisiae* alcohol dehydrogenase/glyceraldehyde-3-phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3-phosphoglycerate kinase. Other useful promoters for yeast host cells are described by Romanos et al., 1992, Yeast 8: 423-488. The control sequence may also be a transcription terminator, which is recognized by a host cell to terminate transcription. The terminator is operably linked to the 3'-terminus of the polynucleotide encoding the polypeptide. Any terminator that is functional in the host cell may be used in the present invention.

Preferred terminators for bacterial host cells are obtained from the genes for *Bacillus clausii* alkaline protease (*aprH*), *Bacillus licheniformis* alpha-amylase (*amyL*), and *Escherichia coli* ribosomal RNA (*rrnB*).

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3-phosphate dehydrogenase. Other useful terminators for yeast host cells are described by Romanos *et al.,* 1992, *supra.*

The control sequence may also be an mRNA stabilizer region downstream of a promoter and upstream of the coding sequence of a gene which increases expression of the gene. Examples of suitable mRNA stabilizer regions are obtained from a *Bacillus thuringiensis cryIIIA* gene (WO 94/25612) and a *Bacillus subtilis* SP82 gene (Hue et al., 1995, Journal of Bacteriology 177: 3465-3471).

The control sequence may also be a leader, a nontranslated region of an mRNA that is important for translation by the host cell. The leader is operably linked to the 5'-terminus of the polynucleotide encoding the polypeptide. Any leader that is functional in the host cell may be used.

Preferred leaders for filamentous fungal host cells are obtained from the genes for *Aspergillus oryzae* TAKA amylase and *Aspergillus nidulans* triose phosphate isomerase.

Suitable leaders for yeast host cells are obtained from the genes for *Saccharomyces cerevisiae* enolase (ENO-1), *Saccharomyces cerevisiae* 3-phosphoglycerate kinase, *Saccharomyces cerevisiae* alpha-factor, and *Saccharomyces cerevisiae* alcohol dehydrogenase/glyceraldehyde-3-phosphate dehydrogenase (ADH2/GAP).

The control sequence may also be a polyadenylation sequence, a sequence operably linked to the 3'-terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Aspergillus nidulans* anthranilate synthase, *Aspergillus niger* glucoamylase, *Aspergillus niger* alpha-glucosidase *Aspergillus oryzae* TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N-terminus of a polypeptide and directs the polypeptide into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (*nprT, nprS, nprM*), and *Bacillus subtilis prsA.* Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*), *Bacillus subtilis* neutral protease (*nprT*), *Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N-terminus of a polypeptide and the signal peptide sequence is positioned next to the N-terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

**Expression Vectors**

The present disclosure also relates to recombinant expression vectors comprising a polynucleotide of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, *i.e.,* a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is an *hph-tk* dual selectable marker system.

The vector preferably contains an element(s) that permits integration of the vector into the host cell's genome or autonomous replication of the vector in the cell independent of the genome. For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate *in vivo.*

Examples of bacterial origins of replication are the origins of replication of plasmids pBR322, pUC19, pACYC177, and pACYC184 permitting replication in *E. coli,* and pUB110, pE194, pTA1060, and pAMβ1 permitting replication in *Bacillus.*

Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.,* Sambrook *et al.,* 1989, *supra*).

**Host Cells**

The present disclosure also relates to recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, *e.g.,* a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any Bacillus cell including, but not limited to, Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis, and Bacillus thuringiensis cells.

The bacterial host cell may also be any Streptococcus cell including, but not limited to, Streptococcus equisimilis, Streptococcus pyogenes, Streptococcus uberis, and Streptococcus equi subsp. Zooepidemicus cells.

The bacterial host cell may also be any Streptomyces cell including, but not limited to, Streptomyces achromogenes, Streptomyces avermitilis, Streptomyces coelicolor, Streptomyces griseus, and Streptomyces lividans cells.

The introduction of DNA into a *Bacillus* cell may be effected by protoplast transformation (see, *e.g.,* Chang and Cohen, 1979, Mol. Gen. Genet. 168: 111-115), competent cell transformation (see, *e.g.,* Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, *e.g.,* Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, *e.g.,* Burke et al., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.,* Catt and Jollick, 1991, Microbios 68: 189-207), electroporation (see, *e.g.,* Buckley et al., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in *Biology and Activities of Yeast* (Skinner, Passmore, and Davenport, editors, Soc. App. Bacteriol. Symposium Series No. 9, 1980). The yeast host cell may be a Candida, Hansenula, Kluyveromyces, Pichia, Saccharomyces, Schizosaccharomyces, or Yarrowia cell, such as a Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis, or Yarrowia lipolytica cell.

The fungal host cell may be a filamentous fungal cell. "Filamentous fungi" include all filamentous forms of the subdivision Eumycota and Oomycota (as defined by Hawksworth *et al.,* 1995, *supra).* The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, or Trichoderma cell.*

*For example, the filamentous fungal host cell may be an Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

## Methods of Production

The present disclosure also relates to methods of producing a polypeptide of the present invention, comprising (a) cultivating a cell, which in its wild-type form produces the polypeptide, under conditions conducive for production of the polypeptide; and optionally, (b) recovering the polypeptide.

The present disclosure also relates to methods of producing a polypeptide of the present invention, comprising (a) cultivating a recombinant host cell of the present invention under conditions conducive for production of the polypeptide; and optionally, (b) recovering the polypeptide.

The host cells are cultivated in a nutrient medium suitable for production of the polypeptide using methods known in the art. For example, the cells may be cultivated by shake flask cultivation, or small-scale or large-scale fermentation (including continuous, batch, fed-batch, or solid state fermentations) in laboratory or industrial fermentors in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (*e.g.,* in catalogues of the American Type Culture Collection). If the polypeptide is secreted into the nutrient medium, the polypeptide can be recovered directly from the medium. If the polypeptide is not secreted, it can be recovered from cell lysates.

The polypeptide may be detected using methods known in the art that are specific for the polypeptides. These detection methods include, but are not limited to, use of specific antibodies, formation of an enzyme product, or disappearance of an enzyme substrate. For example, an enzyme assay may be used to determine the activity of the polypeptide.

The polypeptide may be recovered using methods known in the art. For example, the polypeptide may be recovered from the nutrient medium by conventional procedures including, but not limited to, collection, centrifugation, filtration, extraction, spray-drying, evaporation, or precipitation. In one aspect, a fermentation broth comprising the polypeptide is recovered.

The polypeptide may be purified by a variety of procedures known in the art including, but not limited to, chromatography (*e.g.,* ion exchange, affinity, hydrophobic, chromatofocusing, and size exclusion), electrophoretic procedures (*e.g.,* preparative isoelectric focusing), differential solubility (*e.g.,* ammonium sulfate precipitation), SDS-PAGE, or extraction (see, *e.g.,* Protein Purification, Janson and Ryden, editors, VCH Publishers, New York, 1989) to obtain substantially pure polypeptides.

In an alternative aspect, the polypeptide is not recovered, but rather a host cell of the present invention expressing the polypeptide is used as a source of the polypeptide.

**Fermentation Broth Formulations or Cell Compositions**

The present disclosure also relates to a fermentation broth formulation or a cell composition comprising a polypeptide of the present invention. The fermentation broth product further comprises additional ingredients used in the fermentation process, such as, for example, cells (including, the host cells containing the gene encoding the polypeptide of the present invention which are used to produce the polypeptide of interest), cell debris, biomass, fermentation media and/or fermentation products. In some embodiments, the composition is a cell-killed whole broth containing organic acid(s), killed cells and/or cell debris, and culture medium.

The term "fermentation broth" as used herein refers to a preparation produced by cellular fermentation that undergoes no or minimal recovery and/or purification. For example, fermentation broths are produced when microbial cultures are grown to saturation, incubated under carbon-limiting conditions to allow protein synthesis (*e.g.,* expression of enzymes by host cells) and secretion into cell culture medium. The fermentation broth can contain unfractionated or fractionated contents of the fermentation materials derived at the end of the fermentation. Typically, the fermentation broth is unfractionated and comprises the spent culture medium and cell debris present after the microbial cells (*e.g.,* filamentous fungal cells) are removed, *e.g.,* by centrifugation. In some embodiments, the fermentation broth contains spent cell culture medium, extracellular enzymes, and viable and/or nonviable microbial cells.

In an embodiment, the fermentation broth formulation and cell compositions comprise a first organic acid component comprising at least one 1-5 carbon organic acid and/or a salt thereof and a second organic acid component comprising at least one 6 or more carbon organic acid and/or a salt thereof. In a specific embodiment, the first organic acid component is acetic acid, formic acid, propionic acid, a salt thereof, or a mixture of two or more of the foregoing and the second organic acid component is benzoic acid, cyclohexanecarboxylic acid, 4-methylvaleric acid, phenylacetic acid, a salt thereof, or a mixture of two or more of the foregoing.

In one aspect, the composition contains an organic acid(s), and optionally further contains killed cells and/or cell debris. In one embodiment, the killed cells and/or cell debris are removed from a cell-killed whole broth to provide a composition that is free of these components.

The fermentation broth formulations or cell compositions may further comprise a preservative and/or anti-microbial (*e.g.,* bacteriostatic) agent, including, but not limited to, sorbitol, sodium chloride, potassium sorbate, and others known in the art.

The cell-killed whole broth or composition may contain the unfractionated contents of the fermentation materials derived at the end of the fermentation. Typically, the cell-killed whole broth or composition contains the spent culture medium and cell debris present after the microbial cells (*e.g.,* filamentous fungal cells) are grown to saturation, incubated under carbon-limiting conditions to allow protein synthesis. In some embodiments, the cell-killed whole broth or composition contains the spent cell culture medium, extracellular enzymes, and killed filamentous fungal cells. In some embodiments, the microbial cells present in the cell-killed whole broth or composition can be permeabilized and/or lysed using methods known in the art.

A whole broth or cell composition as described herein is typically a liquid, but may contain insoluble components, such as killed cells, cell debris, culture media components, and/or insoluble enzyme(s). In some embodiments, insoluble components may be removed to provide a clarified liquid composition.

The whole broth formulations and cell compositions of the present invention may be produced by a method described in WO 90/15861 or WO 2010/096673.

**Enzyme Compositions**

The present disclosure also relates to compositions comprising a polypeptide of the present invention. Preferably, the compositions are enriched in such a polypeptide. The term "enriched" indicates that the endo-beta-1,4-mannanase activity of the composition has been increased, *e.g.,* with an enrichment factor of at least 1.1.

The compositions may comprise a polypeptide of the present invention as the major enzymatic component, *e.g.,* a mono-component composition. Alternatively, the compositions may comprise multiple enzymatic activities, such as one or more (*e.g.,* several) enzymes selected from the group consisting of hydrolase, isomerase, ligase, lyase, oxidoreductase, or transferase, *e.g.,* an alpha-galactosidase, alpha-glucosidase, aminopeptidase, amylase, beta-galactosidase, beta-glucosidase, beta-xylosidase, carbohydrase, carboxypeptidase, catalase, cellobiohydrolase, cellulase, chitinase, cutinase, cyclodextrin glycosyltransferase, deoxyribonuclease, endoglucanase, esterase, glucoamylase, invertase, laccase, lipase, mannosidase, mutanase, oxidase, pectinolytic enzyme, peroxidase, phytase, polyphenoloxidase, proteolytic enzyme, ribonuclease, transglutaminase, or xylanase.

The compositions may be prepared in accordance with methods known in the art and may be in the form of a liquid or a dry composition. The compositions may be stabilized in accordance with methods known in the art.

Examples are given below of preferred uses of the compositions of the present invention. The dosage of the composition and other conditions under which the composition is used may be determined on the basis of methods known in the art.

**Uses**

The present disclosure also relates to use of a polypeptide of the invention in coffee extraction. The present invention also relates to a method for producing a coffee extract, comprising the steps:

> a. providing roast and ground coffee beans;
> b. adding to said coffee beans water and a polypeptide of the invention having endo-beta-1,4-mannanase activity;
> c. incubating to make an aqueous coffee extract; and
> d. separating the coffee extract from the extracted coffee beans.

**Examples**

**Enzymes**

In the examples below the following enzymes were used:

| SEQ ID NO: | Example | Description | Origin | GH |
|---|---|---|---|---|
| 1-3 | Example 1 | Endo-$\beta$-1,4-mannanase | *Talaromyces leycettanus* | GH5 |
| 6-8 | Example 2 | Endo-$\beta$-1,4-mannanase | *Chaetomium virescens* | GH5 |
| 11-13 | Example 3 | Endo-$\beta$-1,4-mannanase | *Sordaria macrospora* | GH5 |

**Materials**

Chemicals used as buffers and substrates were commercial products of at least reagent grade.

**Media and Solutions**

YP+2% glucose medium was composed of 1% yeast extract, 2% peptone and 2% glucose.

PDA agar plates were composed of potato infusion (potato infusion was made by boiling 300 g of sliced (washed but unpeeled) potatoes in water for 30 minutes and then decanting or straining the broth through cheesecloth. Distilled water was then added until the total volume of the suspension was one liter, followed by 20 g of dextrose and 20 g of agar powder. The medium was sterilized by autoclaving at 15 psi for 15 minutes (Bacteriological Analytical Manual, 8th Edition, Revision A, 1998).

LB plates were composed of 10 g of Bacto-Tryptone, 5 g of yeast extract, 10 g of sodium chloride, 15 g of Bacto-agar, and deionized water to 1 liter. The medium was sterilized by autoclaving at 15 psi for 15 minutes (Bacteriological Analytical Manual, 8th Edition, Revision A, 1998).

COVE sucrose plates were composed of 342 g Sucrose (Sigma S-9378), 20 g Agar powder, 20 ml Cove salt solution (26 g $MgSO_4.7H_2O$, 26 g KCL, 26 g $KH_2PO_4$, 50 ml Cove trace metal solution) and deionized water to 1 liter), and deionized water to 1 liter). The medium was sterilized by autoclaving at 15 psi for 15 minutes (Bacteriological Analytical Manual, 8th Edition, Revision A, 1998). The medium was cooled to 60°C and added 10 mM acetamide, 15 mM CsCl, Triton X-100 (50 $\mu$l/500 ml)).

Cove trace metal solution was composed of 0.04 g $Na_2B_4O_7.10H_2O$, 0.4 g $CuSO_4.5H_2O$, 1.2 g $FeSO_4.7H_2O$, 0.7 g $MnSO_4.H_2O$, 0.8 g $Na_2MoO_4.2H_2O$, 10 g $ZnSO_4.7H_2O$, and deionized water to 1 liter.

**Example 1: Cloning, expression and purification of the *Talaromyces leycettanus* endo-mannanase (MANNANASE 1)**

**Strains**

*Talaromyces leycettanus* Strain CBS398.68 was used as the source of a polypeptide having mannanase activity. *Aspergillus oryzae* MT3568 strain was used for expression of the *Talaromyces leycettanus* gene encoding the polypeptide having mannanase activity. *A. oryzae* MT3568 is an *amdS* (acetamidase) disrupted gene derivative of *Aspergillus oryzae* JaL355 (WO 02/40694) in which *pyrG* auxotrophy was restored by disrupting the *A. oryzae* acetamidase (*amdS*) gene.

**Source of DNA sequence information for *Talaromyces leycettanus* Strain CBS398.68**

Genomic sequence information was generated by Illumina DNA sequencing at the Beijing Genome Institute (BGI) in Beijing, China from genomic DNA isolated from *Talaromyces leycettanus* Strain CBS398.68. A preliminary assembly of the genome was analyzed using the Pedant-Pro™ Sequence Analysis Suite (Biomax Informatics AG, Martinsried, Germany). Gene models constructed by the software were used as a starting point for detecting GH5 homologues in the genome. More precise gene models were constructed manually using multiple known GH5 protein sequences as a guide.

***Talaromyces leycettanus* Strain CBS398.68 genomic DNA extraction**

To generate genomic DNA for PCR amplification, *Talaromyces leycettanus* Strain CBS398.68 was propagated on PDA agar plates by growing at 26°C for 7 days. Spores harvested from the PDA plates were used to inoculate 25 ml of YP+2% glucose medium in a baffled shake flask and incubated at 26°C for 72 hours with agitation at 85 rpm.

Genomic DNA was isolated according to a modified DNeasy Plant Maxi kit protocol (Qiagen Danmark, Copenhagen, Denmark). The fungal material from the above culture was harvested by centrifugation at 14,000 x g for 2 minutes. The supernatant was removed and the 0.5 g of the pellet was frozen in liquid nitrogen with quartz sand and grinded to a fine powder in a pre-chilled mortar. The powder was transferred to a 15 ml centrifuge tube and added 5 ml buffer AP1 (preheated to 65 °C) and 10 $\mu$l RNase A stock solution (100 mg/ml) followed by vigorous vortexing. After incubation for 10 minutes at 65 °C with regular inverting of the tube, 1.8 ml buffer AP2 was added to the lysate by gentle mixing followed by incubation on ice for 10 min. The lysate was then centrifugated at 3000 x g for 5 minutes at room temperature and the supernatant was decanted into a QIAshredder maxi spin column placed in a 50 ml collection tube. This was followed by centrifugation at 3000 x g for 5 minutes at room temperature. The flow-through was transferred into a new 50 ml tube and added 1.5 volumes of buffer AP3/E followed by vortexing. 15 ml of the sample was transferred into a DNeasy Maxi spin column placed in a 50 ml collection tube and centrifuged at 3000 x g for 5 minutes at room temperature. The flow-through was discarded and 12 ml buffer AW was added to the DNeasy Maxi spin column placed in a 50 ml collection tube and centrifuged at 3000 x g for 10 minutes at room temperature. After discarding the flow-through, centrifugation was repeated to dispose of the remaining alcohol. The DNeasy Maxi spin column was transferred to a new 50 ml tube and 0.5 ml buffer AE (preheated to 70°C) was added. After incubation for 5 minutes at room temperature, the sample was eluted by centrifugation at 3000 x g for 5 minutes at room temperature. Elution was repeated with an additional 0.5 ml buffer AE and the eluates were combined. The concentration of the harvested DNA was measured by a UV spectrophotometer at 260 nm.

**Construction of an *Aspergillus oryzae* expression vector containing *Talaromyces leycettanus* Strain CBS398.68 genomic sequence encoding a Family GH5 polypeptide having mannanase activity**

Two synthetic oligonucleotide primers shown below were designed to PCR amplify the *Talaromyces leycettanus* Strain CBS398.68 P23YST gene (SEQ ID NO: 1) from the genomic DNA prepared as described above. An IN-FUSION™ Cloning Kit (BD Biosciences, Palo Alto, CA, USA) was used to clone the fragment directly into the expression vector pDau109 (WO 2005/042735).
F-P23YST

> 5'-acacaactggggatccaccATGAAGTTGTCTACCCTCAATTTCCT-3' (SEQ ID NO: 4) R-P23YST
> 5'-ccctctagatctcgagCACGTCAGTATCAGCGAAGCAT-3' (SEQ ID NO: 5)

Capital letters represent gene sequence. The underlined sequence is homologous to the insertion sites of pDau109.

An MJ Research PTC-200 DNA engine was used to perform the PCR reaction. A Phusion® High-Fidelity PCR Kit (Finnzymes Oy, Espoo, Finland) was used for the PCR amplification. The PCR reaction was composed of 5 $\mu$l of 5X HF buffer (Finnzymes Oy, Espoo, Finland), 0.5 $\mu$l of dNTPs (10 mM), 0.5 $\mu$l of Phusion® DNA polymerase (0.2 units/$\mu$l) (Finnzymes Oy, Espoo, Finland), 2 $\mu$l of primer F-P23YST (2.5 $\mu$M), 2 $\mu$l of primer R-P23YST (2.5 $\mu$M), 0.5 $\mu$l of *Talaromyces leycettanus* genomic DNA (100 ng/$\mu$l), and 14.5 $\mu$l of deionized water in a total volume of 25 $\mu$l. The PCR conditions were 1 cycle at 95°C for 2 minutes. 35 cycles each at 98°C for 10 seconds, 60°C for 30 seconds, and 72°C for 2 minutes; and 1 cycle at 72°C for 10 minutes. The sample was then held at 12°C until removed from the PCR machine.

The reaction products were isolated by 1.0% agarose gel electrophoresis using 40 mM Tris base, 20 mM sodium acetate, 1 mM disodium EDTA (TAE) buffer where a 1613 bp product band was excised from the gel and purified using an illustra GFX® PCR DNA and Gel Band Purification Kit (GE Healthcare Life Sciences, Brondby, Denmark) according to the manufacturer's instructions. The fragment was then cloned into *Bam* HI and *Xho* I digested pDau109 using an IN-FUSION™ Cloning Kit resulting in plasmid pP23YST. Cloning of the P23YST gene into *Bam* HI-*Xho* I digested pDau109 resulted in the transcription of the *Talaromyces leycettanus* P23YST gene under the control of a NA2-tpi double promoter. NA2-tpi is a modified promoter from the gene encoding the *Aspergillus niger* neutral alpha-amylase in which the untranslated leader has been replaced by an untranslated leader from the gene encoding the *Aspergillus nidulans* triose phosphate isomerase.

The cloning protocol was performed according to the IN-FUSION™ Cloning Kit instructions generating a P23YST GH5 construct. The treated plasmid and insert were transformed into One Shot® TOP10F' Chemically Competent *E. coli* cells (Invitrogen, Carlsbad, CA, USA) according to the manufacturer's protocol and plated onto LB plates supplemented with 0.1 mg of ampicillin per ml. After incubating at 37°C overnight, colonies were seen growing under selection on the LB ampicillin plates. Two colonies transformed with the P23YST GH5 construct were cultivated in LB medium supplemented with 0.1 mg of ampicillin per ml and plasmid was isolated with a QIAprep Spin Miniprep Kit (QIAGEN Inc., Valencia, CA, USA) according to the manufacturer's protocol.

Isolated plasmids were sequenced with vector primers and P23YST gene specific primers in order to determine a representative plasmid expression clone that was free of PCR errors.

**Characterization of the *Talaromyces leycettanus* CBS398.68 genomic sequence encoding a P23YST GH5 polypeptide having mannanase activity**

DNA sequencing of the *Talaromyces leycettanus* CBS398.68 P23YST GH5 genomic clone was performed with an Applied Biosystems Model 3700 Automated DNA Sequencer using version 3.1 BIG-DYE™ terminator chemistry (Applied Biosystems, Inc., Foster City, CA, USA) and primer walking strategy. Nucleotide sequence data were scrutinized for quality and all sequences were compared to each other with assistance of PHRED/PHRAP software (University of Washington, Seattle, WA, USA).

The nucleotide sequence and deduced amino acid sequence of the *Talaromyces leycettanus* P23YST gene is shown in SEQ ID NO: 1 and SEQ ID NO: 2, respectively. The coding sequence is 1548 bp including the stop codon and is interrupted by three introns. The encoded predicted protein is 431 amino acids. Using the SignalP program (Nielsen et al., 1997, Protein Engineering 10: 1-6), a signal peptide of 17 residues was predicted. The predicted mature protein (SEQ ID NO: 3) contains 414 amino acids with a predicted molecular mass of 45 kDa and an isoelectric pH of 4.8. The polypeptide of SEQ ID NO: 3 showed mannanase activity as shown below.

A comparative pairwise global alignment of amino acid sequences was determined using the Needleman and Wunsch algorithm (Needleman and Wunsch, 1970, J. Mol. Biol. 48: 443-453) with gap open penalty of 10, gap extension penalty of 0.5, and the EBLOSUM62 matrix. The alignment showed that the deduced amino acid sequence of the *Talaromyces leycettanus* gene encoding the P23YST GH5 polypeptide having mannanase activity shares 71% identity (excluding gaps) to the deduced amino acid sequence of a predicted GH5 family protein from *Talaromyces stipitatus* (accession number SWISSPROT:B8M6W7) with endo mannanase activity.

**Expression of the Talaromyces leycettanus GH5 mannanase (MANNANASE 1)**

The expression plasmid pP23YST was transformed into *Aspergillus oryzae* MT3568. *Aspergillus oryzae* MT3568 is an AMDS (acetamidase) disrupted derivative of JaL355 (WO 02/40694) in which pyrG auxotrophy was restored in the process of knocking out the *Aspergillus oryzae* acetamidase (AMDS) gene. MT3568 protoplasts are prepared according to the method of European Patent No. 0238023, pages 14-15, which are incorporated herein by reference.

Transformants were purified on COVE sucrose selection plates through single conidia prior to sporulating them on PDA plates. Production of the *Talaromyces leycettanus* GH5 polypeptide by the transformants was analyzed from culture supernatants of 1 ml 96 deep well stationary cultivations at 30°C in YP+2% glucose medium. Expression was verified on an E-Page 8% SDS-PAGE 48 well gel (Invitrogen, Carlsbad, CA, USA) by Coomassie staining. One transformant was selected for further work and designated *Aspergillus oryzae* 11.7.

For larger scale production, *Aspergillus oryzae* 11.7 spores were spread onto a PDA plate and incubated for five days at 37°C. The confluent spore plate was washed twice with 5 ml of 0.01% TWEEN® 20 to maximize the number of spores collected. The spore suspension was then used to inoculate fifteen 500 ml flasks containing 150 ml of Dap-4C medium (WO 2012/103350). The culture was incubated at 30°C with constant shaking at 100 rpm. At day four post-inoculation, the culture broth was collected by filtration through a bottle top MF75 Supor MachV 0.2 μm PES filter (Thermo Fisher Scientific, Roskilde, Denmark). Fresh culture broth from this transformant produced a band of GH5 protein of approximately 42 kDa. The identity of the prominent band as the *Talaromyces leycettanus* GH5 polypeptide was verified by peptide sequencing.

**Alternative method for producing the *Talaromyces leycettanus* GH5 mannanase (MANNANASE 1)**

Based on the nucleotide sequence identified as SEQ ID NO: 1, a synthetic gene can be obtained from a number of vendors such as Gene Art (GENEART AG BioPark, Josef-Engert-Str. 11, 93053, Regensburg, Germany) or DNA 2.0 (DNA2.0, 1430 O'Brien Drive, Suite E, Menlo Park, CA 94025, USA). The synthetic gene can be designed to incorporate additional DNA sequences such as restriction sites or homologous recombination regions to facilitate cloning into an expression vector.

Using the two synthetic oligonucleotide primers F-P23YST and R-P23YST described above, a simple PCR reaction can be used to amplify the full-length open reading frame from the synthetic gene of SEQ ID NO: 1. The gene can then be cloned into an expression vector for example as described above and expressed in a host cell, for example in *Aspergillus oryzae* as described above.

**Purification of the *Talaromyces leycettanus* GH5 mannanase (MANNANASE 1)**

Filtrated broth was adjusted to pH7.0 and filtrated on 0.22$\mu$m PES filter (Nalge Nunc International, Nalgene labware cat#595-4520). Following, the filtrate was added 1.8M ammonium sulphate. The filtrate was loaded onto a Phenyl Sepharose™ 6 Fast Flow column (high sub) (GE Healthcare, Piscataway, NJ, USA) equilibrated with 1.8M ammonium sulphate, 25mM HEPES pH7.0. After wash with 1.0M ammonium sulphate, the bound proteins were batch eluted with 25 mM HEPES pH 7.0. Fractions were collected and analyzed by SDS-PAGE. The fractions were pooled and applied to a Sephadex™ G-25 (medium) (GE Healthcare, Piscataway, NJ, USA) column equilibrated in 25 mM HEPES pH 7.5. The fractions were applied to a SOURCE™ 15Q (GE Healthcare, Piscataway, NJ, USA) column equilibrated in 25 mM HEPES pH 7.5 and bound proteins were eluted with a linear gradient from 0-1000 mM sodium chloride over 20CV. Fractions were collected and analyzed by SDS-PAGE.

**Example 2. Cloning, expression and purification of the *Chaetomium virescens* endo-mannanase (MANNANASE 2)**

**Strains**

*Chaetomium virescens* CBS547.75 was used as the source of a polypeptide having mannanase activity. *Aspergillus oryzae* MT3568 strain was used for expression of the *Chaetomium virescens* gene encoding the polypeptide having mannanase activity. *A. oryzae* MT3568 is an *amdS* (acetamidase) disrupted gene derivative of *Aspergillus oryzae* JaL355 (WO 2002/40694) in which *pyrG* auxotrophy was restored by disrupting the *A. oryzae* acetamidase (amdS) gene.

**Source of DNA sequence information for *Chaetomium virescens* Strain CBS547.75**

Genomic sequence information was generated by Illumina DNA sequencing at The National Center for Genome Resources in Santa Fe, New Mexico from genomic DNA isolated from *Chaetomium virescens* Strain CBS547.75. A preliminary assembly of the genome was analyzed using the Abyss 1.2.0 Sequence Assembler (GSC Software Center, Vancouver, Canada). Gene models constructed by the software were used as a starting point for detecting GH5 homologues in the genome. More precise gene models were constructed manually using multiple known GH5 protein sequences as a guide.

**_Chaetomium virescens_ Strain CBS547.75 genomic DNA extraction**

To generate genomic DNA for PCR amplification, *Chaetomium virescens* Strain CBS547.75 was propagated on PDA agar plates by growing at 26°C for 7 days. Spores harvested from the PDA plates were used to inoculate 25 ml of YP+2% glucose medium in a baffled shake flask and incubated at 26°C for 72 hours with agitation at 85 rpm.

Genomic DNA was isolated according to a modified DNeasy Plant Maxi kit protocol (Qiagen Danmark, Copenhagen, Denmark). The fungal material from the above culture was harvested by centrifugation at 14,000 x g for 2 minutes. The supernatant was removed and the 0.5 g of the pellet was frozen in liquid nitrogen with quartz sand and grinded to a fine powder in a pre-chilled mortar. The powder was transferred to a 15 ml centrifuge tube and added 5 ml buffer AP1 (preheated to 65 °C) and 10 $\mu$l RNase A stock solution (100 mg/ml) followed by vigorous vortexing. After incubation for 10 minutes at 65 °C with regular inverting of the tube, 1.8 ml buffer AP2 was added to the lysate by gentle mixing followed by incubation on ice for 10 min. The lysate was then centrifugated at 3000 x g for 5 minutes at room temperature and the supernatant was decanted into a QIAshredder maxi spin column placed in a 50 ml collection tube. This was followed by centrifugation at 3000 x g for 5 minutes at room temperature. The flow-through was transferred into a new 50 ml tube and added 1.5 volumes of buffer AP3/E followed by vortexing. 15 ml of the sample was transferred into a DNeasy Maxi spin column placed in a 50 ml collection tube and centrifuged at 3000 x g for 5 minutes at room temperature. The flow-through was discarded and 12 ml buffer AW was added to the DNeasy Maxi spin column placed in a 50 ml collection tube and centrifuged at 3000 x g for 10 minutes at room temperature. After discarding the flow-through, centrifugation was repeated to dispose of the remaining alcohol. The DNeasy Maxi spin column was transferred to a new 50 ml tube and 0.5 ml buffer AE (preheated to 70°C) was added. After incubation for 5 minutes at room temperature, the sample was eluted by centrifugation at 3000 x g for 5 minutes at room temperature. Elution was repeated with an additional 0.5 ml buffer AE and the eluates were combined. The concentration of the harvested DNA was measured by a UV spectrophotometer at 260 nm.

**Construction of an Aspergillus oryzae expression vector containing *Chaetomium virescens* Strain CBS547.75 genomic sequence encoding a Family GH5 polypeptide having mannanase activity**

Two synthetic oligonucleotide primers shown below were designed to PCR amplify the *Chaetomium virescens* Strain CBS547.75 P23NUR gene (SEQ ID NO: 6) from the genomic DNA prepared as described above. An IN-FUSION™ Cloning Kit (BD Biosciences, Palo Alto, CA, USA) was used to clone the fragment directly into the expression vector pDau109 (WO 2005/042735).

F-P23NUR
5'-acacaactgggggatccaccATGAAGGCAATCCTCACAGCC-3' (SEQ ID NO: 9)
R-P23NUR
5'-ccctctagatctcgagTGCGTATCACGGGACTTCAGA-3' (SEQ ID NO: 10)

Capital letters represent gene sequence. The underlined sequence is homologous to the insertion sites of pDau109.

An MJ Research PTC-200 DNA engine was used to perform the PCR reaction. A Phusion® High-Fidelity PCR Kit (Finnzymes Oy, Espoo, Finland) was used for the PCR amplification. The PCR reaction was composed of 5 $\mu$l of 5X HF buffer (Finnzymes Oy, Espoo, Finland), 0.5 $\mu$l of dNTPs (10 mM), 0.5 $\mu$l of Phusion® DNA polymerase (0.2 units/$\mu$l) (Finnzymes Oy, Espoo, Finland), 2 $\mu$l of primer F-P23NUR (2.5 $\mu$M), 2 $\mu$l of primer R-P23NUR (2.5 $\mu$M), 0.5 $\mu$l of *Chaetomium virescens* genomic DNA (100 ng/$\mu$l), and 14.5 $\mu$l of deionized water in a total volume of 25 $\mu$l. The PCR conditions were 1 cycle at 95°C for 2 minutes. 35 cycles each at 98°C for 10 seconds, 60°C for 30 seconds, and 72°C for 2.5 minutes; and 1 cycle at 72°C for 10 minutes. The sample was then held at 12°C until removed from the PCR machine.

The reaction products were isolated by 1.0% agarose gel electrophoresis using 40 mM Tris base, 20 mM sodium acetate, 1 mM disodium EDTA (TAE) buffer where a 1288 bp product band was excised from the gel and purified using an illustra GFX® PCR DNA and Gel Band Purification Kit (GE Healthcare Life Sciences, Brondby, Denmark) according to the manufacturer's instructions. The fragment was then cloned into *Bam* HI and *Xho* I digested pDau109 using an IN-FUSION™ Cloning Kit resulting in plasmid pP23NUR. Cloning of the P23NUR gene into *Bam* HI-*Xho* I digested pDau109 resulted in the transcription of the *Chaetomium virescens* P23NUR gene under the control of a NA2-tpi double promoter. NA2-tpi is a modified promoter from the gene encoding the *Aspergillus niger* neutral alpha-amylase in which the untranslated leader has been replaced by an untranslated leader from the gene encoding the *Aspergillus nidulans* triose phosphate isomerase.

The cloning protocol was performed according to the IN-FUSION™ Cloning Kit instructions generating a P23NUR GH5 construct. The treated plasmid and insert were transformed into One Shot® TOP10F' Chemically Competent *E. coli* cells (Invitrogen, Carlsbad, CA, USA) according to the manufacturer's protocol and plated onto LB plates supplemented with 0.1 mg of ampicillin per ml. After incubating at 37°C overnight, colonies were seen growing under selection on the LB ampicillin plates. Four colonies transformed with the P23NUR GH5 construct were cultivated in LB medium supplemented with 0.1 mg of ampicillin per ml and plasmid was isolated with a QIAprep Spin Miniprep Kit (QIAGEN Inc., Valencia, CA, USA) according to the manufacturer's protocol.

Isolated plasmids were sequenced with vector primers and P23NUR gene specific primers in order to determine a representative plasmid expression clone that was free of PCR errors.

**Characterization of the *Chaetomium virescens* CBS547.75 genomic sequence encoding a P23NUR GH5 polypeptide having mannanase activity**

DNA sequencing of the *Chaetomium virescens* CBS547.75 P23NUR GH5 genomic clone was performed with an Applied Biosystems Model 3700 Automated DNA Sequencer using version 3.1 BIG-DYE™ terminator chemistry (Applied Biosystems, Inc., Foster City, CA, USA) and primer walking strategy. Nucleotide sequence data were scrutinized for quality and all sequences were compared to each other with assistance of PHRED/PHRAP software (University of Washington, Seattle, WA, USA).

The nucleotide sequence and deduced amino acid sequence of the *Chaetomium virescens* P23NUR gene is shown in SEQ ID NO: 6 and SEQ ID NO: 7, respectively. The coding sequence is 1222 bp including the stop codon and is interrupted by two introns. The encoded predicted protein is 367 amino acids. Using the SignalP program (Nielsen et al., 1997, Protein Engineering 10: 1-6), a signal peptide of 17 residues was predicted. The predicted mature protein (SEQ ID NO: 8) contains 350 amino acids with a predicted molecular mass of 39 kDa and an isoelectric pH of 6.9. The polypeptide of SEQ ID NO: 8 showed mannanase activity as shown below.

A comparative pairwise global alignment of amino acid sequences was determined using the Needleman and Wunsch algorithm (Needleman and Wunsch, 1970, J. Mol. Biol. 48: 443-453) with gap open penalty of 10, gap extension penalty of 0.5, and the EBLOSUM62 matrix. The alignment showed that the deduced amino acid sequence of the *Chaetomium virescens* gene encoding the P23NUR GH5 polypeptide having mannanase activity shares 86% identity (excluding gaps) to the deduced amino acid sequence of a predicted GH5 family protein from *Chaetomium globosum* (accession number SWISSPROT:Q2H1Y9) with unknown activity.

## Expression of the *Chaetomium virescens* GH5 mannanase P23NUR

The expression plasmid pP23NUR was transformed into *Aspergillus oryzae* MT3568. *Aspergillus oryzae* MT3568 is an AMDS (acetamidase) disrupted derivative of JaL355 (WO 02/40694) in which pyrG auxotrophy was restored in the process of knocking out the *Aspergillus oryzae* acetamidase (AMDS) gene. MT3568 protoplasts are prepared according to the method of European Patent No. 0238023, pages 14-15, which are incorporated herein by reference. Transformants were purified on COVE sucrose selection plates through single conidia prior to sporulating them on PDA plates. Production of the *Chaetomium virescens* GH5 polypeptide by the transformants was analyzed from culture supernatants of 1 ml 96 deep well stationary cultivations at 30°C in YP+2% glucose medium. Expression was verified on an E-Page 8% SDS-PAGE 48 well gel (Invitrogen, Carlsbad, CA, USA) by Coomassie staining. One transformant was selected for further work and designated *Aspergillus oryzae* 29.8.

For larger scale production, *Aspergillus oryzae* 29.8 spores were spread onto a PDA plate and incubated for five days at 37°C. The confluent spore plate was washed twice with 5 ml of 0.01% TWEEN® 20 to maximize the number of spores collected. The spore suspension was then used to inoculate fifteen 500 ml flasks containing 150 ml of Dap-4C medium (WO 2012/103350). The culture was incubated at 30°C with constant shaking at 100 rpm. At day four post-inoculation, the culture broth was collected by filtration through a bottle top MF75 Supor MachV 0.2 $\mu$m PES filter (Thermo Fisher Scientific, Roskilde, Denmark). Fresh culture broth from this transformant produced two bands of GH5 protein of approximately 45 and 50 kDa. The identity of the two bands as the *Chaetomium virescens* GH5 polypeptide was verified by peptide sequencing. The difference between apparent and observed size of the recombinant proteins can likely be attributed to glycosylation and/or other posttranslational modifications.

## Alternative method for producing the *Chaetomium virescens* GH5 mannanase (MANNANASE 2)

Based on the nucleotide sequence identified as SEQ ID NO: 6, a synthetic gene can be obtained from a number of vendors such as Gene Art (GENEART AG BioPark, Josef-Engert-Str. 11, 93053, Regensburg, Germany) or DNA 2.0 (DNA2.0, 1430 O'Brien Drive, Suite E, Menlo Park, CA 94025, USA). The synthetic gene can be designed to incorporate additional DNA sequences such as restriction sites or homologous recombination regions to facilitate cloning into an expression vector.

Using the two synthetic oligonucleotide primers F-P23NUR and R-P23NUR described above, a simple PCR reaction can be used to amplify the full-length open reading frame from the synthetic gene of SEQ ID NO: 4. The gene can then be cloned into an expression vector for example as described above and expressed in a host cell, for example in *Aspergillus oryzae* as described above.

## Purification of the *Chaetomium virescens* endo-mannanase (MANNANASE 2)

Filtrated broth was adjusted to pH7.0 and filtrated on 0.22$\mu$m PES filter (Nalge Nunc International, Nalgene labware cat#595-4520). Following, the filtrate was added 1.8M ammonium sulphate. The filtrate was loaded onto a Phenyl Sepharose™ 6 Fast Flow column (high sub) (GE Healthcare, Piscataway, NJ, USA) equilibrated with 1.8M ammonium sulphate, 25mM HEPES pH7.0. After wash with 1.0M ammonium sulphate, the bound proteins were batch eluted with 25 mM HEPES pH 7.0. Fractions were collected and analyzed by SDS-PAGE. The fractions were pooled and applied to a Sephadex™ G-25 (medium) (GE Healthcare, Piscataway, NJ, USA) column equilibrated in 12.5 mM acetic acid pH 4.3 adjusted with NaOH. The fractions were applied to a SOURCE™ 15S (GE Healthcare, Piscataway, NJ, USA) column equilibrated in 12.5 mM acetic acid pH 4.3/NaOH and bound proteins were eluted with a linear gradient from 0-1000 mM sodium chloride over 20CV. Fractions were collected and analyzed by SDS-PAGE.

**Example 3: Cloning, expression and purification of the *Sordaria macrospora* endo-mannanase (MANNANASE 3)**

**Strains**

*Sordaria macrospora* DSM997 was used as the source of a polypeptide having mannanase activity. *Aspergillus oryzae* MT3568 strain was used for expression of the *Sordaria macrospora* gene encoding the polypeptide having mannanase activity. *A. oryzae* MT3568 is an *amdS* (acetamidase) disrupted gene derivative of *Aspergillus oryzae* JaL355 (WO 2002/40694) in which *pyrG* auxotrophy was restored by disrupting the *A. oryzae* acetamidase (*amdS*) gene.

**Sordaria macrospora Strain DSM997 genomic DNA extraction**

To generate genomic DNA for PCR amplification, *Sordaria macrospora* Strain DSM997 was propagated on PDA agar plates by growing at 26°C for 7 days. Spores harvested from the PDA plates were used to inoculate 25 ml of YP+2% glucose medium in a baffled shake flask and incubated at 26°C for 72 hours with agitation at 85 rpm.

Genomic DNA was isolated according to a modified DNeasy Plant Maxi kit protocol (Qiagen Danmark, Copenhagen, Denmark). The fungal material from the above culture was harvested by centrifugation at 14,000 x g for 2 minutes. The supernatant was removed and the 0.5 g of the pellet was frozen in liquid nitrogen with quartz sand and grinded to a fine powder in a pre-chilled mortar. The powder was transferred to a 15 ml centrifuge tube and added 5 ml buffer AP1 (preheated to 65 °C) and 10 μl RNase A stock solution (100 mg/ml) followed by vigorous vortexing. After incubation for 10 minutes at 65 °C with regular inverting of the tube, 1.8 ml buffer AP2 was added to the lysate by gentle mixing followed by incubation on ice for 10 min. The lysate was then centrifugated at 3000 x g for 5 minutes at room temperature and the supernatant was decanted into a QIAshredder maxi spin column placed in a 50 ml collection tube. This was followed by centrifugation at 3000 x g for 5 minutes at room temperature. The flow-through was transferred into a new 50 ml tube and added 1.5 volumes of buffer AP3/E followed by vortexing.

15 ml of the sample was transferred into a DNeasy Maxi spin column placed in a 50 ml collection tube and centrifuged at 3000 x g for 5 minutes at room temperature. The flow-through was discarded and 12 ml buffer AW was added to the DNeasy Maxi spin column placed in a 50 ml collection tube and centrifuged at 3000 x g for 10 minutes at room temperature. After discarding the flow-through, centrifugation was repeated to dispose of the remaining alcohol. The DNeasy Maxi spin column was transferred to a new 50 ml tube and 0.5 ml buffer AE (preheated to 70°C) was added. After incubation for 5 minutes at room temperature, the sample was eluted by centrifugation at 3000 x g for 5 minutes at room temperature. Elution was repeated with an additional 0.5 ml buffer AE and the eluates were combined. The concentration of the harvested DNA was measured by a UV spectrophotometer at 260 nm.

**Construction of an *Aspergillus oryzae* expression vector containing *Sordaria macrospora* Strain DSM997 genomic sequence encoding a Family GH5 polypeptide having mannanase activity**

Two synthetic oligonucleotide primers shown below were designed to PCR amplify the *Sordaria macrospora* Strain DSM997 P2453A gene (SEQ ID NO: 11) from the genomic DNA prepared as described above. P2453A correspond to the genome sequence of SwissProt entry D1ZM91, annotated as a GH5 putative cellulase. An IN-FUSION™ Cloning Kit (BD Biosciences, Palo Alto, CA, USA) was used to clone the fragment directly into the expression vector pDau109 (WO 2005/042735).

F-P2453A
5'-acacaactgggggatccaccATGAAGTCCTTGTTCACCCTCGCC-3' (SEQ ID NO: 14)
R-P2453A
5'-ccctctagatctcgagGTACGCAGCCACGGCGACA-3' (SEQ ID NO: 15)

Capital letters represent gene sequence. The underlined sequence is homologous to the insertion sites of pDau109.
An MJ Research PTC-200 DNA engine was used to perform the PCR reaction. A Phusion® High-Fidelity PCR Kit (Finnzymes Oy, Espoo, Finland) was used for the PCR amplification. The PCR reaction was composed of 5 μl of 5X HF buffer (Finnzymes Oy, Espoo, Finland), 0.5 μl of dNTPs (10 mM), 0.5 μl of Phusion® DNA polymerase (0.2 units/μl) (Finnzymes Oy, Espoo, Finland), 2 μl of primer F-P2453A (2.5 μM), 2 μl of primer R-P2453A (2.5 μM), 0.5 μl of *Sordaria macrospora* genomic DNA (100 ng/μl), and 14.5 μl of deionized water in a total volume of 25 μl. The PCR conditions were 1 cycle at 95°C for 2.5 minutes. 35 cycles each at 98°C for 10 seconds, 60°C for 30 seconds, and 72°C for 2.5 minutes; and 1 cycle at 72°C for 10 minutes. The sample was then held at 12°C until removed from the PCR machine.
The reaction products were isolated by 1.0% agarose gel electrophoresis using 40 mM Tris base, 20 mM sodium acetate, 1 mM disodium EDTA (TAE) buffer where a 1260 bp product band was excised from the gel and purified using an illustra GFX® PCR DNA and Gel Band Purification Kit (GE Healthcare Life Sciences, Brondby, Denmark) according to the manufacturer's instructions. The fragment was then cloned into *Bam* HI and *Xho* I digested pDau109 using an IN-FUSION™ Cloning Kit resulting in plasmid pP2453A. Cloning of the P2453A gene into *Bam* HI-*Xho* I digested pDau109 resulted in the transcription of the *Sordaria macrospora* P2453A gene under the control of a NA2-tpi double promoter. NA2-tpi is a modified promoter from the gene encoding the *Aspergillus niger* neutral alpha-amylase in which the untranslated leader has been replaced by an untranslated leader from the gene encoding the *Aspergillus nidulans* triose phosphate isomerase.

The cloning protocol was performed according to the IN-FUSION™ Cloning Kit instructions generating a P2453A GH5 construct. The treated plasmid and insert were transformed into One Shot® TOP10F' Chemically Competent *E. coli* cells (Invitrogen, Carlsbad, CA, USA) according to the manufacturer's protocol and plated onto LB plates supplemented with 0.1 mg of ampicillin per ml. After incubating at 37°C overnight, colonies were seen growing under selection on the LB ampicillin plates. Two colonies transformed with the P2453A GH5 construct were cultivated in LB medium supplemented with 0.1 mg of ampicillin per ml and plasmid was isolated with a QIAprep Spin Miniprep Kit (QIAGEN Inc., Valencia, CA, USA) according to the manufacturer's protocol.

Isolated plasmids were sequenced with vector primers and P2453A gene specific primers in order to determine a representative plasmid expression clone that was free of PCR errors.

**Characterization of the *Sordaria macrospora* DSM997 genomic sequence encoding a P2453A GH5 polypeptide having mannanase activity**

DNA sequencing of the *Sordaria macrospora* DSM997 P2453A GH5 genomic clone was performed with an Applied Biosystems Model 3700 Automated DNA Sequencer using version 3.1 BIG-DYE™ terminator chemistry (Applied Biosystems, Inc., Foster City, CA, USA) and primer walking strategy. Nucleotide sequence data were scrutinized for quality and all sequences were compared to each other with assistance of PHRED/PHRAP software (University of Washington, Seattle, WA, USA).

The nucleotide sequence and deduced amino acid sequence of the *Sordaria macrospora* P2453A gene is shown in SEQ ID NO: 11 and SEQ ID NO: 12, respectively. The coding sequence is 1203 bp including the stop codon and is interrupted by two introns. The encoded predicted protein is 361 amino acids. Using the SignalP program (Nielsen et al., 1997, Protein Engineering 10: 1-6), a signal peptide of 17 residues was predicted. The predicted mature protein contains 344 amino acids (SEQ ID NO: 13) with a predicted molecular mass of 38 kDa and an isoelectric pH of 6.4. The polypeptide of SEQ ID NO: 13 showed mannanase activity as shown below.

A comparative pairwise global alignment of amino acid sequences was determined using the Needleman and Wunsch algorithm (Needleman and Wunsch, 1970, J. Mol. Biol. 48: 443-453) with gap open penalty of 10, gap extension penalty of 0.5, and the EBLOSUM62 matrix. The alignment showed that the deduced amino acid sequence of the *Sordaria macrospora* gene encoding the P2453A GH5 polypeptide having mannanase activity shares 77% identity (excluding gaps) to the deduced amino acid sequence of a predicted GH5 family protein from *Chaetomium globosum* (accession number SWISSPROT:Q2H1Y9) with unknown activity.

**Expression of the *Sordaria macrospora* GH5 mannanase (MANNANASE 3)**

The expression plasmid pP2453A was transformed into *Aspergillus oryzae* MT3568. *Aspergillus oryzae* MT3568 is an AMDS (acetamidase) disrupted derivative of JaL355 (WO 02/40694) in which pyrG auxotrophy was restored in the process of knocking out the *Aspergillus oryzae* acetamidase (AMDS) gene. MT3568 protoplasts are prepared according to the method of European Patent No. 0238023, pages 14-15, which are incorporated herein by reference.

Transformants were purified on COVE sucrose selection plates through single conidia prior to sporulating them on PDA plates. Production of the *Sordaria macrospora* GH5 polypeptide by the transformants was analyzed from culture supernatants of 1 ml 96 deep well stationary cultivations at 30°C in YP+2% glucose medium. Expression was verified on an E-Page 8% SDS-PAGE 48 well gel (Invitrogen, Carlsbad, CA, USA) by Coomassie staining. One transformant was selected for further work and designated *Aspergillus oryzae* 46.7.

For larger scale production, *Aspergillus oryzae* 46.7 spores were spread onto a PDA plate and incubated for five days at 37°C. The confluent spore plate was washed twice with 5 ml of 0.01% TWEEN® 20 to maximize the number of spores collected. The spore suspension was then used to inoculate fifteen 500 ml flasks containing 150 ml of Dap-4C medium (WO 2012/103350). The culture was incubated at 30°C with constant shaking at 100 rpm. At day four post-inoculation, the culture broth was collected by filtration through a bottle top MF75 Supor MachV 0.2 μm PES filter (Thermo Fisher Scientific, Roskilde, Denmark). Fresh culture broth from this transformant produced two bands of GH5 protein of approximately 47 and 50 kDa. The identity of the two bands as the *Sordaria macrospora* GH5 polypeptide was verified by peptide sequencing. The difference between apparent and observed size of the recombinant proteins can likely be attributed to glycosylation and/or other posttranslational modifications.

**Alternative method for producing the *Sordaria macrospora* GH5 mannanase (MANNANASE 3)**

Based on the nucleotide sequence identified as SEQ ID NO: 11, a synthetic gene can be obtained from a number of vendors such as Gene Art (GENEART AG BioPark, Josef-Engert-Str. 11, 93053, Regensburg, Germany) or DNA 2.0 (DNA2.0, 1430 O'Brien Drive, Suite E, Menlo Park, CA 94025, USA). The synthetic gene can be designed to incorporate additional DNA sequences such as restriction sites or homologous recombination regions to facilitate cloning into an expression vector.

Using the two synthetic oligonucleotide primers F-P2453A and R-P2453A described above, a simple PCR reaction can be used to amplify the full-length open reading frame from the synthetic gene of SEQ ID NO: 7. The gene can then be cloned into an expression vector for example as described above and expressed in a host cell, for example in *Aspergillus oryzae* as described above.

**Purification of the *Sordaria macrospora* endo-mannanase (MANNANASE 3)**

Filtrated broth was adjusted to pH7.0 and filtrated on 0.22$\mu$m PES filter (Nalge Nunc International, Nalgene labware cat#595-4520). Following, the filtrate was added 1.8M ammonium sulphate. The filtrate was loaded onto a Phenyl Sepharose™ 6 Fast Flow column (high sub) (GE Healthcare, Piscataway, NJ, USA) equilibrated with 1.8M ammonium sulphate, 25mM HEPES pH7.0. After wash with 1.0M ammonium sulphate, the bound proteins were batch eluted with 12.5 mM HEPES pH 7.0. Fractions were collected and analyzed by SDS-PAGE. The fractions were pooled and applied to a Sephadex™ G-25 (medium) (GE Healthcare, Piscataway, NJ, USA) column equilibrated in 25 mM HEPES pH 7.0. The fractions were applied to a SOURCE™ 15Q (GE Healthcare, Piscataway, NJ, USA) column equilibrated in 12.5 mM HEPES pH 7.5 and bound proteins were eluted with a linear gradient from 0-1000 mM sodium chloride over 20CV. Fractions were collected and analyzed by SDS-PAGE. The protein was recovered in the effluent.

**Example 4: Thermostability of mannanases evaluated by DSC**

MANNANASE 4 used in this and some of the following examples is a GH5_8 mannanase originally obtained from *Caldicellulosiruptor saccharolyticus* and having an amino acid sequence represented by the mature amino acid sequence of SEQ ID NO: 17. The mature amino acid sequence has been determined as amino acids 28-319 by N-terminal sequencing and mass spectrometry (MS) of the full-length protein. The mature amino acid sequence is shown as SEQ ID NO: 18.

Thermostabilities of MANNANASE 1, MANNANASE 2, MANNANASE 3 and MANNANASE 4 were evaluated by Differential Scanning Calorimetry (DSC) in the appropriate buffer solution (20 mM Sodium acetate pH 5). The temperature corresponding to the apex of the peak in the thermogram was noted as the thermal transition midpoint ($T_m(°C)$) for the enzymes.

Table 1: Midpoint temperatures

| Enzyme | Temperature (°C) |
| --- | --- |
| MANNANASE 1 | 93 |
| MANNANASE 2 | 69 |
| MANNANASE 3 | 74 |
| MANNANASE 4 | 92 |

For comparison, the thermal transition midpoint for Mannaway determined by DSC at pH 5 is 73°C. The thermal transition midpoint for Gamanase (beta-mannanase from *Aspergillus niger*) determined by DSC at pH 5 is 87°C. And the thermal transition midpoint for beta-mannanase from *Trichoderma reesei* used in Example 10 determined by DSC at pH 5 is 81°C.

**Example 5: Mannanase activity on AZCL-galactomannan**

Activity of the mannanases were assayed by the hydrolysis of 0.2 w/v% AZCL-galactomannan in 50mM Britton-Robinson Buffer (50mM phosphoric acid, 50mM acetic acid, 50mM boric acid, 50mM KCl, 1mM $CaCl_2$) and 0.01% Triton X-100, pH 5 at 40°C for 10 min. Experimental mannanases and Mannaway® 25L were added individually to give a final concentration of 0-0.01 mg/ml. The reactions were terminated on an ice/water bath. After centrifugation (10,000rpm, 5 min at 4°C), the supernatants were transferred to a microtiter plate and the absorbance at 595 nm was measured. The procedures were performed in triplicates for all enzymes and a blank (no enzyme). For all 4 enzymes a dose response could be observed (Table 2).

Table 2. Absorbance at 600 nm.

| Conc. (mg/mL) | Mannaway $A_{600}$ | MANNANASE 1 $A_{600}$ | MANNANASE 2 $A_{600}$ | MANNANASE3 $A_{600}$ |
|---|---|---|---|---|
| 0.0100 | 0.529 | 1.201 | 0.463 | 0.294 |
| 0.0075 | 0.405 | 1.034 | 0.381 | 0.233 |
| 0.0050 | 0.282 | 0.811 | 0.295 | 0.163 |
| 0.0025 | 0.156 | 0.535 | 0.178 | 0.087 |
| 0.0010 | 0.070 | 0.257 | 0.082 | 0.037 |
| 0.0000 | 0.000 | 0.000 | 0.000 | 0.000 |

**Example 6: Pretreatment of coffee material**

800 mL boiling water was added to 155 g roasted and grinded Arabica coffee beans with a particle size of 0.5 mm. After incubation in a water bath at 95°C for 30 min with manual mixing every 5 min, the slurry was cooled down at room temperature. After an initial vacuum filtration through a Whatman GF/D filter, $\varnothing$ 150 mm, the insoluble spent coffee on the filter was washed by adding 500 - 1000 mL MilliQ water. The spent coffee was removed from the filter and spread out on a large sheet and left to dry overnight. The spent coffee grounds were further defatted by water saturated butanol. The butanol fraction was separated by filtration and the defatted spent coffee grounds were dried under vacuum before use. This defatted spent coffee grounds were used in Example 7.

**Example 7: Enzyme catalyzed hydrolysis of defatted spent coffee grounds**

**a) Enzymatic extraction**

Defatted spent coffee grounds produced according to Example 6 (10 weight %) was incubated with water and a suitably diluted enzyme (to give a final reaction concentration of 1.47 nM for MANNANASE 1-4 and 0.2% Mannaway® 25L) at 50°C. Samples were withdrawn after 2 and 24 hours and the enzymatic hydrolysis was stopped immediately by heating the samples at 100°C for 10 min. After centrifugation (10,000×g, 10 min) and filtration through a 0.22 $\mu$m filter, the supernatants were further analyzed for dry matter, carbohydrate composition and absorbance. The procedures were performed in duplicate for all enzymes and a blank (no enzyme added).

**b) Dry matter determination**

Dry matter (DM) content was quantified after overnight drying at 110°C of supernatants from enzyme treated spent coffee grounds. The weight of the dry matter was divided by the added volume of supernatant and a DM value based on g/L was calculated. The characteristics of the extract based on DM are summarized in Table 3.

Table 3: Dry matter of defatted spent grounds extract after different enzymatic treatments.

| Enzyme treatment | Dry matter (g /L) | |
|---|---|---|
| | 2h | 24h |
| No enzyme | 2.8 | 2.5 |
| Mannaway | 6.1 | 7.2 |
| MANNANASE 1 | 10.0 | 16.1 |
| MANNANASE 2 | 9.6 | 11.3 |
| MANNANASE 3 | 8.0 | 9.1 |
| MANNANASE 4 | 8.1 | 9.2 |

All experimental mannanases solubilize more dry matter than Mannaway, both after 2 hrs and 24 hrs incubation time (Table 3).

**c) Carbohydrate analysis**

The sugar composition was analysed by measuring free monosaccharides in the supernatants by high-performance anion exchange chromatography with pulsed amperometric detection (HPAEC-PAD). The total sugars were analysed by HPAEC-PAD after acid hydrolysis in 2 M trifluoro acetic acid for 2 h at 95°C. The acid hydrolysed samples were neutralised by an initial dilution in 0.2 M NaOH. Monosaccharides were quantified after suitable dilutions against a 5-point standard curve of arabinose (Ara), galactose (Gal), glucose (Glc) and mannose (Man) between 0.002-0.02 g/L. The results can be seen in Table 4, Table 5 and Table 6.

Table 4: Free monosaccharides in the extract from defatted spent coffee grounds after enzymatic treatment.

| | Free monosaccharides/DM (%) | | | |
|---|---|---|---|---|
| | 2h | | 24h | |
| Enzyme treatment | Glu | Man | Glu | Man |
| No enzyme | 0 | 0 | 0 | 0 |
| Mannaway | 0 | 1.5 | 0 | 1.5 |
| MANNANASE 1 | 0.2 | 2.0 | 0.2 | 4.6 |
| MANNANASE 2 | 0 | 1.6 | 0 | 2.3 |
| MANNANASE 3 | 0 | 0.4 | 0 | 0.5 |

Table 5: Total sugar composition in the extract from defatted spent coffee grounds after enzymatic treatment. Monosaccharides in supernatant after acid hydrolysis.

| | Total monosaccharides/DM (%) | | | | | | | |
|---|---|---|---|---|---|---|---|---|
| | 2h | | | | 24h | | | |
| Enzyme treatment | Ara | Gal | Glu | Man | Ara | Gal | Glu | Man |
| No enzyme | 0.7 | 2.4 | 0.8 | 11.0 | 1.7 | 6.1 | 0.9 | 15.2 |
| Mannaway | 0.7 | 2.7 | 1.1 | 19.6 | 0.9 | 3.9 | 0.6 | 21.3 |
| MANNANASE 1 | 0.5 | 2.4 | 1.3 | 18.7 | 0.6 | 3.2 | 0.4 | 20.0 |
| MANNANASE 2 | 0.5 | 2.5 | 1.1 | 18.9 | 0.7 | 3.5 | 0.4 | 20.4 |
| MANNANASE 3 | 0.5 | 2.6 | 1.1 | 18.3 | 0.8 | 3.6 | 0.4 | 19.8 |

Table 6: Percentage of the saccharides present as monosaccharides based on the weight of the total sugars as monosaccharides.

| | Free monosaccharides/ Total sugars (%) | |
|---|---|---|
| Enzyme treatment | 2h | 24h |
| No enzyme | 0 | 0 |
| Mannaway | 6 | 6 |
| MANNANASE 1 | 10 | 20 |
| MANNANASE 2 | 7 | 9 |
| MANNANASE 3 | 2 | 2 |

**d) Absorbance**

The absorbance at 361 nm of samples was measured after suitable dilutions of supernatants and alkalinisation by at least a 1:10 dilution in 0.2 M $Na_2CO_3$. Dividing the absorbance by the DM (g/L) gave a quality measurement relating to released colour by DM (Table 7). The mannanases released similar colour per DM as Mannaway.

Table 7: Quality of extract. Absorbance of extract after alkalinisation at 361 nm per dry matter.

| Enzyme treatment | Absorbance ($A_{361}$*L/g) | |
| --- | --- | --- |
| | 2h | 24h |
| Mannaway | 0.04 | 0.05 |
| MANNANASE 1 | 0.04 | 0.04 |
| MANNANASE 2 | 0.04 | 0.03 |
| MANNANASE 3 | 0.04 | 0.04 |

## Example 8: Enzyme catalyzed hydrolysis of defatted spent coffee grounds

The enzymatic solubilisation of spent defatted coffee grounds were performed with Mannaway and MANNANASE4 using the method describe in Example 7. The difference was that two temperatures were tested for the enzymatic extraction, 50°C and 80°C, and dry matter was measured on the resulting supernatants.

Table 8. Dry matter of defatted spent grounds extract after different enzymatic treatments at 50°C and 80°C

| Enzyme treatment | Dry matter (g/L) | | | |
| --- | --- | --- | --- | --- |
| | 50°C | | 80°C | |
| | 2h | 24h | 2h | 24h |
| No enzyme | 1.2 | 0.5 | 1.4 | 3.2 |
| Mannaway | 5.8 | 6.7 | 4.6 | 3.6 |
| MANNANASE 4 | 8.1 | 9.2 | 8.8 | 11.4 |

Table 8 clearly shows that using a thermostable mannanase enables higher solubilisation temperatures and achieves higher solubilisation degrees at equal enzyme dosing.

## Example 9: Enzyme catalyzed hydrolysis of spent coffee grounds

### Preparation of spent coffee

Roasted Arabica beans (238 g) were milled using a 1 mm sieve and the resulting milled fraction was extracted with boiling water at a dry matter of 20 %. The temperature after mixing was 87 °C. The spent coffee grounds were separated from the liquid by vacuum filtration using Whatman GF/D filters after 10 min of mixing. The spent coffee was washed with an excess of water before drying over night at 60 °C. Based on the dry matter in the filtrate the partition of the dry matter was 25 % in the liquid phase and 75 % in the solid phase.

### Enzymatic hydrolysis of the spent coffee produced

Spent coffee grounds produced as described above was incubated at 10 weight% with water and a suitably diluted mannanase (to give a final reaction concentration of 50 mg/L for MANNANASE 1, MANNANASE 4 and 0.2 volume % Mannaway® 25L) at 50, 70, 80 and 90 °C. Samples were heat inactivated at 100 °C for 10 min after 2 or 24 hours enzymatic hydrolysis. After centrifugation (10,000×g, 10 min) and filtration through a 0.22 $\mu$m filter, the supernatants were analyzed for dry matter. The procedures were performed in duplicate for all enzymes and a blank (no enzyme added).
Dry matter was measured as described in Example 7.

Table 9. The effect of temperature and time on the solubilization-degree of spent coffee

| Treatment | 2 hrs | | | | 24 hrs | | | |
| --- | --- | --- | --- | --- | --- | --- | --- | --- |
| | 50°C | 70°C | 80°C | 90°C | 50°C | 70°C | 80°C | 90°C |
| No enzyme | 1%[1] | 2% | 2% | 3% | 3% | 4% | 5% | 7% |
| Mannaway® 25 L | 4% | 3% | 2% | 3% | 7% | 4% | 5% | 8% |
| MANNANASE1 | 4% | 5% | 5% | 3% | 9% | 12% | 10% | 8% |

(continued)

| Treatment | 2 hrs | | | | 24 hrs | | | |
|---|---|---|---|---|---|---|---|---|
| | 50°C | 70°C | 80°C | 90°C | 50°C | 70°C | 80°C | 90°C |
| MANNANASE 4 | 6% | 9% | 10% | 1%[1] | 14% | 21% | 17% | 10% |
| [1] Standard deviation above 1 percentage point | | | | | | | | |

Mannaway could solubilize some of the spent coffee grounds at 50°C in both the short and long incubation time but at temperatures at or above 70°C there was no significant solubilization compared to the untreated sample. For MANNANASE 1 and MANNANASE 4 the solubilization optimum for the longer enzyme incubation was 70°C and at the shorter incubation time 70-80°C was the optimal temperature range (Table 9). MANNANASE 1 and MANNANASE 4 could therefore be used at higher temperature where significantly increased extraction yields were observed and hence lead to better process economy.

**Example 10:**

Coffee grounds prepared according to Example 6 was used at a final assay concentration of 10% dry matter and hydrolysed for 2 or 24 hrs at 55°C on a thermomixer at 1200 rpm. The enzyme concentration was 0.5 mg enzyme per kg spent coffee grounds. The enzyme was inactivated by boiling for 10 min and the supernatant transferred to a separate tube after 10 min centrifugation at 10,000 rfc. Approximately 0.75 g extract was taken out and the liquid evaporated at 105°C before the dry matter was recorded.

Gamanase is beta-mannanase from *Aspergillus niger*. "T. reesei + CBM1" is beta-mannanase from *Trichoderma reesei* including a CBM1 binding domain having the amino acid sequence shown as amino acids 1 to 418 of SEQ ID NO: 19. "T. reesei - CBM1" is beta-mannanase from *Trichoderma reesei* without the CBM1 binding domain having the amino acid sequence shown as amino acids 1 to 355 of SEQ ID NO: 20.

Table 10. Enzymatic solubilization of defatted coffee grounds compared to a blank with no enzyme added

| | 2 hrs | 24 hrs |
|---|---|---|
| **No enzyme added** | 1.1±0.2% | 3.0±0.1% |
| **Mannaway** | 2.2±0.1% | 2.7±0.2% |
| **MANNANASE 1** | 7.5±0.1% | 14.7±0.2% |
| **Gamanase** | 1.9±0.1% | 5.2±0.1% |
| *T. reesei* + **CBM1** | 4.5±0.3% | 10.3±0.2% |
| *T. reesei* - **CBM1** | 2.9±0% | 8.1±0.2% |

**Claims**

1. A method for producing a coffee extract, comprising the steps:

   a. providing roast and ground coffee beans;
   b. optionally performing one or more first extractions of said coffee beans;
   c. adding to said coffee beans, which have optionally been subjected to one or more first extractions, water and an enzyme having mannanase activity;
   d. incubating to make an aqueous coffee extract; and
   e. separating the coffee extract from the extracted coffee beans,

   wherein the enzyme having mannanase activity has at least 70% sequence identity to SEQ ID NO: 3.

2. The method of claim 1, wherein the enzyme having mannanase activity has at least 75%, at least 80%, at least 85%, at least 90%, at least 95%, at least 96%, at least 97%, at least 98%, at least 99% or 100% sequence identity to SEQ ID NO: 3.

3. The method of any of claims 1-2, wherein the enzyme having mannanase activity is thermostable.

4. The method of any of claims 1-3, wherein the enzyme having mannanase activity has a melting temperature ($T_m$) determined by Differential Scanning Calorimetry (DSC) of at least 80°C, preferably at least 85°C or at least 90°C.

5. The method of any of claims 1-4, wherein the incubation in step d is performed for at least one hour.

6. The method of any of claims 1-5, wherein the incubation in step d. is performed at a temperature of at least 60°C such as at least 65°C, preferably at least 70°C such as at least 75°C or at least 80°C.

7. The method of any of the preceding claims, wherein the enzyme having mannanase activity is an endo-beta-1,4-mannanase.

8. The method of any of the preceding claims, wherein the enzyme having mannanase activity is a GH5 endo-beta-1,4-mannanase, preferably a GH5_7 endo-beta-1,4-mannanase or a GH5_8 endo-beta-1,4-mannanase.

9. The method of any of the preceding claims, wherein the roast and ground coffee beans are subjected to one or more first extractions before step c.

10. The method of claim 9, wherein a steam explosion is performed after the first extraction and before step c.

11. The method of any of claims 9 or 10, wherein a second milling of the coffee beans is performed after the first extraction and before step c.

12. The method of any of the preceding claims, wherein the coffee extract obtained in step e comprises at least 100% more dry matter than a coffee extract prepared by a similar method without the addition of an enzyme having mannanase activity.

13. The method of any of claims 9-12, wherein at least 8% by weight of the dry matter of the partially extracted coffee beans obtained after step b is recovered in the coffee extract obtained in step e.

**Patentansprüche**

1. Verfahren zur Herstellung eines Kaffeeextrakts, umfassend die Schritte:

   a. Bereitstellen gerösteter und gemahlener Kaffeebohnen;
   b. gegebenenfalls Durchführen einer oder mehrerer erster Extraktionen der Kaffeebohnen;
   c. Versetzen der Kaffeebohnen, die gegebenenfalls einer oder mehreren ersten Extraktionen unterzogen wurden, mit Wasser und einem Enzym mit Mannanase-Aktivität;
   d. Inkubieren zur Erzeugung eines wässrigen Kaffeeextrakts; und
   e. Trennen des Kaffeeextrakts von den extrahierten Kaffeebohnen,

   wobei das Enzym mit Mannanase-Aktivität eine Sequenzidentität von wenigstens 70% mit SEQ ID NO: 3 aufweist.

2. Verfahren nach Anspruch 1, wobei das Enzym mit Mannanase-Aktivität eine Sequenzidentität von wenigstens 75%, wenigstens 80%, wenigstens 85%, wenigstens 90%, wenigstens 95%, wenigstens 96%, wenigstens 97%, wenigstens 98%, wenigstens 99% oder 100% mit SEQ ID NO: 3 aufweist.

3. Verfahren nach einem der Ansprüche 1-2, wobei das Enzym mit Mannanase-Aktivität thermostabil ist.

4. Verfahren nach einem der Ansprüche 1-3, wobei das Enzym mit Mannanase-Aktivität eine mit DSC (Differential Scanning Calorimetry) bestimmte Schmelztemperatur ($T_m$) von wenigstens 80°C, bevorzugt wenigstens 85°C oder wenigstens 90°C.

5. Verfahren nach einem der Ansprüche 1-4, wobei die Inkubation in Schritt d wenigstens eine Stunde lang durchgeführt wird.

6. Verfahren nach einem der Ansprüche 1-5, wobei die Inkubation in Schritt d. bei einer Temperatur von wenigstens 60°C, z. B. wenigstens 65°C, bevorzugt wenigstens 70°C, z. B. wenigstens 75°C oder wenigstens 80°C durchgeführt wird.

7. Verfahren nach einem der vorhergehenden Ansprüche, wobei es sich bei dem Enzym mit Mannanase-Aktivität um eine Endo-beta-1,4-Mannanase handelt.

8. Verfahren nach einem der vorhergehenden Ansprüche, wobei es sich bei dem Enzym mit Mannanase-Aktivität um eine GH5-Endo-beta-1,4-Mannanase, bevorzugt eine GH5_7-Endo-beta-1,4-Mannanase oder eine GH5_8-Endo-beta-1,4-Mannanase handelt.

9. Verfahren nach einem der vorhergehenden Ansprüche, wobei die gerösteten und gemahlenen Kaffeebohnen einer oder mehreren ersten Extraktionen vor Schritt c unterzogen werden.

10. Verfahren nach Anspruch 9, wobei nach der ersten Extraktion und vor Schritt c eine Dampfexplosion durchgeführt wird.

11. Verfahren nach einem der Ansprüche 9 oder 10, wobei nach der ersten Extraktion und vor Schritt c ein zweites Mahlen der Kaffeebohnen durchgeführt wird.

12. Verfahren nach einem der vorhergehenden Ansprüche, wobei der in Schritt e erhaltene Kaffeeextrakt wenigstens 100% mehr Trockenmasse als ein mit einem analogen Verfahren ohne die Zugabe eines Enzyms mit Mannanase-Aktivität hergestellter Kaffeeextrakt umfasst.

13. Verfahren nach einem der Ansprüche 9-12, wobei wenigstens 8 Gew.-% der Trockenmasse der nach Schritt b erhaltenen teilextrahierten Kaffeebohnen im in Schritt e erhaltenen Kaffeeextrakt wiedergewonnen werden.

**Revendications**

1. Méthode de production d'un extrait de café, comprenant les étapes consistant à :

   a. mettre à disposition des grains de café torréfiés et moulus ;
   b. éventuellement, mettre en œuvre une ou plusieurs premières extractions desdits grains de café ;
   c. ajouter auxdits grains de café, qui ont été éventuellement soumis à une ou plusieurs premières extractions, de l'eau et une enzyme ayant une activité de mannanase ;
   d. incuber afin de produire un extrait de café aqueux ; et
   e. séparer l'extrait de café et les grains de café extraits ;

   dans laquelle l'enzyme ayant une activité de mannanase possède au moins 70% d'identité de séquence avec SEQ ID n° 3.

2. Méthode selon la revendication 1, dans laquelle l'enzyme ayant une activité de mannanase possède au moins 75%, au moins 80%, au moins 85%, au moins 90%, au moins 95%, au moins 96%, au moins 97%, au moins 98%, au moins 99%, ou 100%, d'identité de séquence avec SEQ ID n° 3.

3. Méthode selon l'une quelconque des revendications 1-2, dans laquelle l'enzyme ayant une activité de mannanase est thermostable.

4. Méthode selon l'une quelconque des revendications 1-3, dans laquelle l'enzyme ayant une activité de mannanase possède une température de fusion ($T_m$), déterminée par Calorimétrie Différentielle à Balayage (DSC), d'au moins 80°C, préférablement d'au moins 85°C ou d'au moins 90°C.

5. Méthode selon l'une quelconque des revendications 1-4, dans laquelle l'incubation dans l'étape d est mise en œuvre pendant au moins une heure.

6. Méthode selon l'une quelconque des revendications 1-5, dans laquelle l'incubation dans l'étape d est mise en œuvre à une température d'au moins 60°C, tel que d'au moins 65°C, préférablement d'au moins 70°C, tel que d'au moins 75°C, ou d'au moins 80°C.

7. Méthode selon l'une quelconque des revendications précédentes, dans laquelle l'enzyme ayant une activité de mannanase est une endo-bêta-1,4-mannanase.

8. Méthode selon l'une quelconque des revendications précédentes, dans laquelle l'enzyme ayant une activité de mannanase est une GH5 endo-bêta-1,4-mannanase, préférablement une GH5_7 endo-bêta-1,4-mannanase ou une GH5_8 endo-bêta-1,4-mannanase.

9. Méthode selon l'une quelconque des revendications précédentes, dans laquelle les grains de café torréfiés et moulus sont soumis à une ou plusieurs premières extractions avant l'étape c.

10. Méthode selon la revendication 9, dans laquelle une explosion de vapeur est mise en œuvre après la première extraction et avant l'étape c.

11. Méthode selon l'une quelconque des revendications 9 ou 10, dans laquelle une deuxième mouture des grains de café est mise en œuvre après la première extraction et avant l'étape c.

12. Méthode selon l'une quelconque des revendications précédentes, dans laquelle l'extrait de café obtenu dans l'étape e comprend au moins 100% de plus de matière sèche par rapport à un extrait de café préparé par une méthode similaire sans addition d'une enzyme ayant une activité de mannanase.

13. Méthode selon l'une quelconque des revendications 9-12, dans laquelle au moins 8% en poids de la matière sèche des grains de café partiellement extraits obtenue après l'étape b est récupérée dans l'extrait de café obtenu dans l'étape e.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4983408 A
- WO 2007011531 A
- US 5714183 A
- EP 2052078 B1
- US 2009325240 A1
- CN 103525792 A
- EP 0489401 A
- WO 9517413 A
- WO 9522625 A
- US 5223409 A
- WO 9206204 A
- WO 9943835 A
- WO 9600787 A
- WO 0056900 A

- US 6011147 A
- WO 9425612 A
- WO 9533836 A
- WO 2010039889 A
- WO 0024883 A
- EP 238023 A
- WO 9015861 A
- WO 2010096673 A
- WO 0240694 A
- EP 0238023 A
- WO 2012103350 A
- WO 200240694 A
- WO 2005042735 A

**Non-patent literature cited in the description**

- **STAALBRAND et al.** Purification and characterization of two beta-mannanases from Trichoderma reesei. *J. Biotechnol.,* 1993, vol. 29, 229-42
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **RICE et al.** *EMBOSS: The European Molecular Biology Open Software Suite,* 2000
- **SAMBROOK et al.** Molecular Cloning, A Laboratory Manual. Cold Spring Harbor, 1989
- **H. NEURATH ; R.L. HILL.** The Proteins. Academic Press, 1979
- **CUNNINGHAM ; WELLS.** *Science,* 1989, vol. 244, 1081-1085
- **HILTON et al.** *J. Biol. Chem.,* 1996, vol. 271, 4699-4708
- **VOS et al.** *Science,* vol. 255, 306-312
- **SMITH et al.** *J. Mol. Biol.,* 1992, vol. 224, 899-904
- **WLODAVER et al.** *FEBS Lett.,* 1992, vol. 309, 59-64
- **REIDHAAR-OLSON ; SAUER.** *Science,* 1988, vol. 241, 53-57
- **BOWIE ; SAUER.** *Proc. Natl. Acad. Sci. USA,* 1989, vol. 86, 2152-2156
- **LOWMAN et al.** *Biochemistry,* 1991, vol. 30, 10832-10837
- **DERBYSHIRE et al.** *Gene,* 1986, vol. 46, 145
- **NER et al.** *DNA,* 1988, vol. 7, 127

- **NESS et al.** *Nature Biotechnology,* 1999, vol. 17, 893-896
- **COOPER et al.** *EMBO J.,* 1993, vol. 12, 2575-2583
- **DAWSON et al.** *Science,* 1994, vol. 266, 776-779
- **MARTIN et al.** *J. Ind. Microbiol. Biotechnol.,* 2003, vol. 3, 568-576
- **SVETINA et al.** *J. Biotechnol.,* 2000, vol. 76, 245-251
- **RASMUSSEN-WILSON et al.** *Appl. Environ. Microbiol.,* 1997, vol. 63, 3488-3493
- **WARD et al.** *Biotechnology,* 1995, vol. 13, 498-503
- **CONTRERAS et al.** *Biotechnology,* 1991, vol. 9, 378-381
- **EATON et al.** *Biochemistry,* 1986, vol. 25, 505-512
- **COLLINS-RACIE et al.** *Biotechnology,* 1995, vol. 13, 982-987
- **CARTER et al.** *Proteins: Structure, Function, and Genetics,* 1989, vol. 6, 240-248
- **STEVENS.** *Drug Discovery World,* 2003, vol. 4, 35-48
- **INNIS et al.** PCR: A Guide to Methods and Application. Academic Press, 1990
- **FORD et al.** *Protein Expression and Purification,* 1991, vol. 2, 95-107
- **AGAISSE ; LERECLUS.** *Molecular Microbiology,* 1994, vol. 13, 97-107
- **EGON et al.** *Gene,* 1988, vol. 69, 301-315

- **VILLA-KAMAROFF** et al. *Proc. Natl. Acad. Sci. USA,* 1978, vol. 75, 3727-3731
- **DEBOER** et al. *Proc. Natl. Acad. Sci. USA,* 1983, vol. 80, 21-25
- **GILBERT** et al. *Scientific American,* 1980, vol. 242, 74-94
- **ROMANOS** et al. *Yeast,* 1992, vol. 8, 423-488
- **HUE** et al. *Journal of Bacteriology,* 1995, vol. 177, 3465-3471
- **GUO ; SHERMAN.** *Mol. Cellular Biol.,* 1995, vol. 15, 5983-5990
- **SIMONEN ; PALVA.** *Microbiological Reviews,* 1993, vol. 57, 109-137
- **GEMS** et al. *Gene,* 1991, vol. 98, 61-67
- **CULLEN** et al. *Nucleic Acids Res.,* 1987, vol. 15, 9163-9175
- **CHANG ; COHEN.** *Mol. Gen. Genet.,* 1979, vol. 168, 111-115
- **YOUNG ; SPIZIZEN.** *J. Bacteriol.,* 1961, vol. 81, 823-829
- **DUBNAU ; DAVIDOFF-ABELSON.** *J. Mol. Biol.,* 1971, vol. 56, 209-221
- **SHIGEKAWA ; DOWER.** *Biotechniques,* 1988, vol. 6, 742-751
- **KOEHLER ; THORNE.** *J. Bacteriol.,* 1987, vol. 169, 5271-5278
- **HANAHAN.** *J. Mol. Biol.,* 1983, vol. 166, 557-580
- **DOWER** et al. *Nucleic Acids Res.,* 1988, vol. 16, 6127-6145
- **GONG** et al. *Folia Microbiol. (Praha),* 2004, vol. 49, 399-405
- **MAZODIER** et al. *J. Bacteriol.,* 1989, vol. 171, 3583-3585
- **BURKE** et al. *Proc. Natl. Acad. Sci. USA,* 2001, vol. 98, 6289-6294
- **CHOI** et al. *J. Microbiol. Methods,* 2006, vol. 64, 391-397
- **PINEDO ; SMETS.** *Appl. Environ. Microbiol.,* 2005, vol. 71, 51-57
- **PERRY ; KURAMITSU.** *Infect. Immun.,* 1981, vol. 32, 1295-1297
- **CATT ; JOLLICK.** *Microbios,* 1991, vol. 68, 189-207
- **BUCKLEY** et al. *Appl. Environ. Microbiol.,* 1999, vol. 65, 3800-3804
- **CLEWELL.** *Microbiol. Rev.,* 1981, vol. 45, 409-436
- **HAWKSWORTH** et al. Ainsworth and Bisby's Dictionary of The Fungi. CAB International, University Press
- Soc. App. Bacteriol. Symposium Series. 1980
- **YELTON** et al. *Proc. Natl. Acad. Sci. USA,* 1984, vol. 81, 1470-1474
- **CHRISTENSEN** et al. *Bio/Technology,* 1988, vol. 6, 1419-1422
- **MALARDIER** et al. *Gene,* 1989, vol. 78, 147-156
- **BECKER ; GUARENTE.** Guide to Yeast Genetics and Molecular Biology, Methods in Enzymology. Academic Press, Inc, vol. 194, 182-187
- **ITO** et al. *J. Bacteriol.,* 1983, vol. 153, 163
- **HINNEN** et al. *Proc. Natl. Acad. Sci. USA,* 1978, vol. 75, 1920
- Protein Purification. VCH Publishers, 1989
- Bacteriological Analytical Manual. 1998
- **NIELSEN** et al. *Protein Engineering,* 1997, vol. 10, 1-6

SEQUENCE LISTING

<110> Novozymes A/S

<120> METHOD FOR PRODUCING A COFFEE EXTRACT

<130> 12696-WO-PCT

<160> 20

<170> PatentIn version 3.5

<210> 1
<211> 1548
<212> DNA
<213> Talaromyces leycettanus

<220>
<221> CDS
<222> (1)..(169)

<220>
<221> Intron
<222> (170)..(263)

<220>
<221> CDS
<222> (264)..(971)

<220>
<221> Intron
<222> (972)..(1053)

<220>
<221> CDS
<222> (1054)..(1182)

<220>
<221> Intron
<222> (1183)..(1258)

<220>
<221> CDS
<222> (1259)..(1545)

<400> 1

```
atg aag ttg tct acc ctc aat ttc ctg tcc ttg gcc ggt ctg gtg tct        48
Met Lys Leu Ser Thr Leu Asn Phe Leu Ser Leu Ala Gly Leu Val Ser
1               5                   10                  15

gcc cag gtt gcc aac tat ggc caa tgt ggt gga cag aat tat tct ggc        96
Ala Gln Val Ala Asn Tyr Gly Gln Cys Gly Gly Gln Asn Tyr Ser Gly
                20                  25                  30

ccg aca act tgc aat ccg ggc tgg tct tgc caa tat ctg aat cca tat       144
Pro Thr Thr Cys Asn Pro Gly Trp Ser Cys Gln Tyr Leu Asn Pro Tyr
            35                  40                  45

tat agc cag tgt ctt cca gct acc c gtatgtcgac tacactcatg                189
Tyr Ser Gln Cys Leu Pro Ala Thr
        50                  55
```

```
tgcatatcag actctgatgt tcccatccgc ttttggtact acattcttgt ttccttgcta          249


attcatcaac acag aa  acg acc act ctg acg acg tcg acg aag ccc acc            298
             Gln Thr Thr Thr Leu Thr Thr Ser Thr Lys Pro Thr
                      60                  65

agc acc agc acc acc acc aga acc agt acc agt acc acc agc acc cag            346
Ser Thr Ser Thr Thr Thr Arg Thr Ser Thr Ser Thr Thr Ser Thr Gln
    70              75                  80

ggc ggc tcg tca agc aca tct ata ccc agc aag aat ggt ctc aag ttt            394
Gly Gly Ser Ser Ser Thr Ser Ile Pro Ser Lys Asn Gly Leu Lys Phe
85              90                  95                  100

acc att gac ggc aag acc gcc tac tat gca ggc acc aac acc tac tgg            442
Thr Ile Asp Gly Lys Thr Ala Tyr Tyr Ala Gly Thr Asn Thr Tyr Trp
            105             110             115

ctc ccg ttc ctg acc aac aat gcg gat gtt gat ctg gtc atg agc cat            490
Leu Pro Phe Leu Thr Asn Asn Ala Asp Val Asp Leu Val Met Ser His
        120             125             130

ctc caa caa tcc ggc ctc aag atc ctt cgt gtc tgg ggc ttc aac gac            538
Leu Gln Gln Ser Gly Leu Lys Ile Leu Arg Val Trp Gly Phe Asn Asp
        135             140             145

gtc aac acc cag cca gga agt ggc acc gtg tgg ttc cag ctg ctc cag            586
Val Asn Thr Gln Pro Gly Ser Gly Thr Val Trp Phe Gln Leu Leu Gln
    150             155             160

aac ggc cag gcg act atc aac acg ggc gcc aat ggt cta cag cgc ctc            634
Asn Gly Gln Ala Thr Ile Asn Thr Gly Ala Asn Gly Leu Gln Arg Leu
165             170             175             180

gac tac gtg gtg caa tct gcg gaa gct cac gat atc aaa ctg atc att            682
Asp Tyr Val Val Gln Ser Ala Glu Ala His Asp Ile Lys Leu Ile Ile
                185             190             195

aac ttt gtc aac aac tgg aac gat tat ggc ggc atc aac gcg tac gtc            730
Asn Phe Val Asn Asn Trp Asn Asp Tyr Gly Gly Ile Asn Ala Tyr Val
            200             205             210

aat aac tat ggc ggt aat gca acg acc tgg tac acc aac tcg gcc gct            778
Asn Asn Tyr Gly Gly Asn Ala Thr Thr Trp Tyr Thr Asn Ser Ala Ala
            215             220             225

cag gct gcg tat cgt aac tac atc aag gcg gtc atc tct cgg tac att            826
Gln Ala Ala Tyr Arg Asn Tyr Ile Lys Ala Val Ile Ser Arg Tyr Ile
    230             235             240

ggc tct cct gcg atc ttt gct tgg gag ttg gcc aat gag ccc cgc tgc            874
Gly Ser Pro Ala Ile Phe Ala Trp Glu Leu Ala Asn Glu Pro Arg Cys
245             250             255             260

cat ggg tgc gac acc tct gtg atc tac aac tgg gtc tct agc acc agt            922
His Gly Cys Asp Thr Ser Val Ile Tyr Asn Trp Val Ser Ser Thr Ser
            265             270             275

gca tac atc aag tct ctt gag cca aac cgc atg gtc tgc atc gga gat g          971
Ala Tyr Ile Lys Ser Leu Glu Pro Asn Arg Met Val Cys Ile Gly Asp
            280             285             290

gtaagtcccc cctccgggga gctcgagatg acaaactcga aacccatgat tcaatcaaaa          1031
```

35

```
ctaacattcg taatctgttc ag ag  ggc atg ggt ctc acc acc gga tcc gac        1082
                             Glu Gly Met Gly Leu Thr Thr Gly Ser Asp
                                     295                 300


ggc agt tat ccc ttc caa tac acc gaa ggt acc gac ttc gag aag aac        1130
Gly Ser Tyr Pro Phe Gln Tyr Thr Glu Gly Thr Asp Phe Glu Lys Asn
        305                 310                 315

ctg gcc atc ccc acc att gat ttc ggc acc ctg cac ttg tac cct agc        1178
Leu Ala Ile Pro Thr Ile Asp Phe Gly Thr Leu His Leu Tyr Pro Ser
        320                 325                 330

agc t gtaagtcaaa gcctcttttc cagtccatat gcatacacag aaccccttcc         1232
Ser
335

actgactcgt acttttctcc gaatag gg  ggc gaa caa gac tcc tgg ggc agc        1284
                              Trp Gly Glu Gln Asp Ser Trp Gly Ser
                                              340

acc tgg atc tcc gcc cac ggc caa gca tgc gtc aat gcc ggc aag ccc        1332
Thr Trp Ile Ser Ala His Gly Gln Ala Cys Val Asn Ala Gly Lys Pro
345                 350                 355                 360

tgc ctc ctg gaa gaa tat gga tcc acc aat cac tgc tct tcc gaa gct        1380
Cys Leu Leu Glu Glu Tyr Gly Ser Thr Asn His Cys Ser Ser Glu Ala
                365                 370                 375

ccc tgg cag tcg acc gct ctc agc acg aac ggt atc gcg gct gac agt        1428
Pro Trp Gln Ser Thr Ala Leu Ser Thr Asn Gly Ile Ala Ala Asp Ser
                380                 385                 390

ttc tgg cag tac ggt gat acc tta agc acg ggc cag tcg ccg aat gac        1476
Phe Trp Gln Tyr Gly Asp Thr Leu Ser Thr Gly Gln Ser Pro Asn Asp
                395                 400                 405

ggg tat acc att tac tac ggt agc agc gat tat acc tgc ttg gtg acg        1524
Gly Tyr Thr Ile Tyr Tyr Gly Ser Ser Asp Tyr Thr Cys Leu Val Thr
        410                 415                 420

aat cat att agc cag ttt cag tga                                        1548
Asn His Ile Ser Gln Phe Gln
425                 430
```

<210> 2
<211> 431
<212> PRT
<213> Talaromyces leycettanus

<400> 2

```
Met Lys Leu Ser Thr Leu Asn Phe Leu Ser Leu Ala Gly Leu Val Ser
1               5                   10                  15

Ala Gln Val Ala Asn Tyr Gly Gln Cys Gly Gly Gln Asn Tyr Ser Gly
            20                  25                  30

Pro Thr Thr Cys Asn Pro Gly Trp Ser Cys Gln Tyr Leu Asn Pro Tyr
            35                  40                  45
```

```
Tyr Ser Gln Cys Leu Pro Ala Thr Gln Thr Thr Thr Leu Thr Thr Ser
    50              55              60

Thr Lys Pro Thr Ser Thr Ser Thr Thr Thr Arg Thr Ser Thr Ser Thr
    65              70              75              80

Thr Ser Thr Gln Gly Gly Ser Ser Ser Thr Ser Ile Pro Ser Lys Asn
            85              90              95

Gly Leu Lys Phe Thr Ile Asp Gly Lys Thr Ala Tyr Tyr Ala Gly Thr
            100             105             110

Asn Thr Tyr Trp Leu Pro Phe Leu Thr Asn Asn Ala Asp Val Asp Leu
            115             120             125

Val Met Ser His Leu Gln Gln Ser Gly Leu Lys Ile Leu Arg Val Trp
    130             135             140

Gly Phe Asn Asp Val Asn Thr Gln Pro Gly Ser Gly Thr Val Trp Phe
145             150             155             160

Gln Leu Leu Gln Asn Gly Gln Ala Thr Ile Asn Thr Gly Ala Asn Gly
            165             170             175

Leu Gln Arg Leu Asp Tyr Val Val Gln Ser Ala Glu Ala His Asp Ile
            180             185             190

Lys Leu Ile Ile Asn Phe Val Asn Asn Trp Asn Asp Tyr Gly Gly Ile
            195             200             205

Asn Ala Tyr Val Asn Asn Tyr Gly Gly Asn Ala Thr Thr Trp Tyr Thr
210             215             220

Asn Ser Ala Ala Gln Ala Ala Tyr Arg Asn Tyr Ile Lys Ala Val Ile
225             230             235             240

Ser Arg Tyr Ile Gly Ser Pro Ala Ile Phe Ala Trp Glu Leu Ala Asn
            245             250             255

Glu Pro Arg Cys His Gly Cys Asp Thr Ser Val Ile Tyr Asn Trp Val
            260             265             270

Ser Ser Thr Ser Ala Tyr Ile Lys Ser Leu Glu Pro Asn Arg Met Val
            275             280             285

Cys Ile Gly Asp Glu Gly Met Gly Leu Thr Thr Gly Ser Asp Gly Ser
```

38

290                              295                              300

Tyr Pro Phe Gln Tyr Thr Glu Gly Thr Asp Phe Glu Lys Asn Leu Ala
305                 310             315                 320

Ile Pro Thr Ile Asp Phe Gly Thr Leu His Leu Tyr Pro Ser Ser Trp
                325             330                 335

Gly Glu Gln Asp Ser Trp Gly Ser Thr Trp Ile Ser Ala His Gly Gln
                340             345                 350

Ala Cys Val Asn Ala Gly Lys Pro Cys Leu Leu Glu Glu Tyr Gly Ser
                355             360                 365

Thr Asn His Cys Ser Ser Glu Ala Pro Trp Gln Ser Thr Ala Leu Ser
    370             375                 380

Thr Asn Gly Ile Ala Ala Asp Ser Phe Trp Gln Tyr Gly Asp Thr Leu
385             390                 395                 400

Ser Thr Gly Gln Ser Pro Asn Asp Gly Tyr Thr Ile Tyr Tyr Gly Ser
                405             410                 415

Ser Asp Tyr Thr Cys Leu Val Thr Asn His Ile Ser Gln Phe Gln
        420             425                 430

<210> 3
<211> 414
<212> PRT
<213> Talaromyces leycettanus

<400> 3

39

Gln Val Ala Asn Tyr Gly Gln Cys Gly Gly Gln Asn Tyr Ser Gly Pro
1                   5                   10                  15

Thr Thr Cys Asn Pro Gly Trp Ser Cys Gln Tyr Leu Asn Pro Tyr Tyr
            20                  25                  30

Ser Gln Cys Leu Pro Ala Thr Gln Thr Thr Thr Leu Thr Thr Ser Thr
            35                  40                  45

Lys Pro Thr Ser Thr Ser Thr Thr Thr Arg Thr Ser Thr Ser Thr Thr
    50                  55                  60

Ser Thr Gln Gly Gly Ser Ser Ser Thr Ser Ile Pro Ser Lys Asn Gly
65                  70                  75                  80

Leu Lys Phe Thr Ile Asp Gly Lys Thr Ala Tyr Tyr Ala Gly Thr Asn

```
              85                    90                    95


     Thr Tyr Trp Leu Pro Phe Leu Thr Asn Asn Ala Asp Val Asp Leu Val
             100               105               110

     Met Ser His Leu Gln Gln Ser Gly Leu Lys Ile Leu Arg Val Trp Gly
             115               120               125

     Phe Asn Asp Val Asn Thr Gln Pro Gly Ser Gly Thr Val Trp Phe Gln
             130               135               140

     Leu Leu Gln Asn Gly Gln Ala Thr Ile Asn Thr Gly Ala Asn Gly Leu
     145               150               155               160

     Gln Arg Leu Asp Tyr Val Val Gln Ser Ala Glu Ala His Asp Ile Lys
                     165               170               175

     Leu Ile Ile Asn Phe Val Asn Asn Trp Asn Asp Tyr Gly Gly Ile Asn
                 180               185               190

     Ala Tyr Val Asn Asn Tyr Gly Gly Asn Ala Thr Thr Trp Tyr Thr Asn
                 195               200               205

     Ser Ala Ala Gln Ala Ala Tyr Arg Asn Tyr Ile Lys Ala Val Ile Ser
         210               215               220

     Arg Tyr Ile Gly Ser Pro Ala Ile Phe Ala Trp Glu Leu Ala Asn Glu
     225               230               235               240

     Pro Arg Cys His Gly Cys Asp Thr Ser Val Ile Tyr Asn Trp Val Ser
                 245               250               255

     Ser Thr Ser Ala Tyr Ile Lys Ser Leu Glu Pro Asn Arg Met Val Cys
                 260               265               270

     Ile Gly Asp Glu Gly Met Gly Leu Thr Thr Gly Ser Asp Gly Ser Tyr
                 275               280               285

     Pro Phe Gln Tyr Thr Glu Gly Thr Asp Phe Glu Lys Asn Leu Ala Ile
         290               295               300

     Pro Thr Ile Asp Phe Gly Thr Leu His Leu Tyr Pro Ser Ser Trp Gly
     305               310               315               320

     Glu Gln Asp Ser Trp Gly Ser Thr Trp Ile Ser Ala His Gly Gln Ala
                 325               330               335
```

```
Cys Val Asn Ala Gly Lys Pro Cys Leu Leu Glu Glu Tyr Gly Ser Thr
            340                 345                 350

Asn His Cys Ser Ser Glu Ala Pro Trp Gln Ser Thr Ala Leu Ser Thr
            355                 360                 365

Asn Gly Ile Ala Ala Asp Ser Phe Trp Gln Tyr Gly Asp Thr Leu Ser
            370                 375                 380

Thr Gly Gln Ser Pro Asn Asp Gly Tyr Thr Ile Tyr Tyr Gly Ser Ser
385                 390                 395                 400

Asp Tyr Thr Cys Leu Val Thr Asn His Ile Ser Gln Phe Gln
                405                 410
```

<210> 4
<211> 45
<212> DNA
<213> Artificial Sequence

<220>
<223> Primer

<400> 4
acacaactgg ggatccacca tgaagttgtc taccctcaat ttcct 45

<210> 5
<211> 38
<212> DNA
<213> Artificial Sequence

<220>
<223> Primer

<400> 5
ccctctagat ctcgagcacg tcagtatcag cgaagcat 38

<210> 6
<211> 1222
<212> DNA
<213> Chaetomium virescens

<220>
<221> CDS
<222> (1)..(799)

<220>
<221> Intron
<222> (800)..(859)

<220>
<221> CDS
<222> (860)..(949)

<220>

<221> Intron
<222> (950)..(1007)

<220>
<221> CDS
<222> (1008)..(1219)

<400> 6

<221> Intron
<222> (950)..(1007)

```
atg aag gca atc ctc aca gcc ggg ctc gga ctg ctc tcc gca gtc cag     48
Met Lys Ala Ile Leu Thr Ala Gly Leu Gly Leu Leu Ser Ala Val Gln
1               5              10             15

gct ctt ccc tcg gcg aag gct gcc tct gcc acc acc aac ggc act cgc     96
Ala Leu Pro Ser Ala Lys Ala Ala Ser Ala Thr Thr Asn Gly Thr Arg
                20             25             30

ttc acc gtc gac ggc aag acg ggc tac ttc gcg ggt acc aat tcg tac    144
Phe Thr Val Asp Gly Lys Thr Gly Tyr Phe Ala Gly Thr Asn Ser Tyr
        35             40             45

tgg atc ggc ttc ctg acc aac aac aag gac atc gac acc act ctg gac    192
Trp Ile Gly Phe Leu Thr Asn Asn Lys Asp Ile Asp Thr Thr Leu Asp
    50             55             60

cac atc tcg tcc tct ggt ctc aag atc ctg cgc gtc tgg ggc ttc aac    240
His Ile Ser Ser Ser Gly Leu Lys Ile Leu Arg Val Trp Gly Phe Asn
65             70             75             80

gac gtc aac acc aag ccc agc gac ggc act gtc tgg tac cag ctc ctc    288
Asp Val Asn Thr Lys Pro Ser Asp Gly Thr Val Trp Tyr Gln Leu Leu
                85             90             95

tcc ccg tcc ggt tca aag atc aac acg ggt gcc gac ggc ctg cag cgg    336
Ser Pro Ser Gly Ser Lys Ile Asn Thr Gly Ala Asp Gly Leu Gln Arg
            100            105            110

ctc gac cat gta gtc aag tcc gct gag aag cgc ggc gtc aag ctg atc    384
Leu Asp His Val Val Lys Ser Ala Glu Lys Arg Gly Val Lys Leu Ile
        115            120            125

atc aac ttc gtc aac aac tgg gac gat tac ggc ggc atg aac gcc tac    432
Ile Asn Phe Val Asn Asn Trp Asp Asp Tyr Gly Gly Met Asn Ala Tyr
        130            135            140

gtc aag gcc ttc ggc ggc acc aag gag ggt tgg tac acc aac gcc aag    480
Val Lys Ala Phe Gly Gly Thr Lys Glu Gly Trp Tyr Thr Asn Ala Lys
145            150            155            160

gct cag cag cag tac aag aag tac atc aag gcc gtg gtc agc cgc tat    528
Ala Gln Gln Gln Tyr Lys Lys Tyr Ile Lys Ala Val Val Ser Arg Tyr
                165            170            175

gcc aag tcg cca gcc gtg ttt gcc tgg gag ctg gcg aac gag ccc cgc    576
Ala Lys Ser Pro Ala Val Phe Ala Trp Glu Leu Ala Asn Glu Pro Arg
            180            185            190

tgc aag gga tgc agc acc gat gtc atc tac aag tgg gcg acc gag atc    624
Cys Lys Gly Cys Ser Thr Asp Val Ile Tyr Lys Trp Ala Thr Glu Ile
            195            200            205

tcg gcg tac atc cgc aag ctg gat ccg agc cac atg atc acg ctc ggt    672
Ser Ala Tyr Ile Arg Lys Leu Asp Pro Ser His Met Ile Thr Leu Gly
210            215            220
```

```
gat gag ggc ttt ggc ctg cct ggt gac acg acc tac ccg tac agc tac        720
Asp Glu Gly Phe Gly Leu Pro Gly Asp Thr Thr Tyr Pro Tyr Ser Tyr
225                 230                 235                 240

acc gag ggt gtc gat ttt gtc aag aac ttg ggc atc aag aac ttg gac        768
Thr Glu Gly Val Asp Phe Val Lys Asn Leu Gly Ile Lys Asn Leu Asp
                    245                 250                 255

ttt gga aca ttc cat atg tat ccc gac agc t gtgcgttgac tccccgtccc        819
Phe Gly Thr Phe His Met Tyr Pro Asp Ser
                260                 265

ttcccccatc tattaccttt gagactgaca ggggagaaag gg  ggc gtc cca tac        873
                                                Trp Gly Val Pro Tyr
                                                            270

agc ttc ggc gag ggg tgg atc aag aac cat gcc gcg gct tgc aag cca        921
Ser Phe Gly Glu Gly Trp Ile Lys Asn His Ala Ala Ala Cys Lys Pro
                275                 280                 285

gcc ggc aag cct tgt ctt ttg gag gag t gtacgttcca ctaccagccc           969
Ala Gly Lys Pro Cys Leu Leu Glu Glu
                290                 295

tttcccagcc catgagcgaa atctgacagc ccgtgcag at   ggt gcc gaa cac agc    1024
                                              Tyr Gly Ala Glu His Ser
                                                            300

tgc gac atc cag aag ccc tgg cag cag gcc tcg ctc gcg ctc gcc aag      1072
Cys Asp Ile Gln Lys Pro Trp Gln Gln Ala Ser Leu Ala Leu Ala Lys
            305                 310                 315

gag ggc atg tcg gga gac ctc ttc tgg caa tgg ggt gac gcc ctg agc      1120
Glu Gly Met Ser Gly Asp Leu Phe Trp Gln Trp Gly Asp Ala Leu Ser
            320                 325                 330

ttc ggc cag tca ccc aac gac ggc cac acg gtc tac tac ggc tcg gag      1168
Phe Gly Gln Ser Pro Asn Asp Gly His Thr Val Tyr Tyr Gly Ser Glu
335                 340                 345                 350

ctt gct caa tgc ctg gtt aca gat cat gtt aag gag att aat gct tct      1216
Leu Ala Gln Cys Leu Val Thr Asp His Val Lys Glu Ile Asn Ala Ser
                355                 360                 365

tcg taa                                                              1222
Ser
```

<210> 7
<211> 367
<212> PRT
<213> Chaetomium virescens

<400> 7

```
Met Lys Ala Ile Leu Thr Ala Gly Leu Gly Leu Leu Ser Ala Val Gln
1               5                   10                  15


Ala Leu Pro Ser Ala Lys Ala Ala Ser Ala Thr Thr Asn Gly Thr Arg
            20                  25                  30
```

```
Phe Thr Val Asp Gly Lys Thr Gly Tyr Phe Ala Gly Thr Asn Ser Tyr
        35              40              45


Trp Ile Gly Phe Leu Thr Asn Asn Lys Asp Ile Asp Thr Thr Leu Asp
    50              55              60


His Ile Ser Ser Ser Gly Leu Lys Ile Leu Arg Val Trp Gly Phe Asn
65              70              75              80


Asp Val Asn Thr Lys Pro Ser Asp Gly Thr Val Trp Tyr Gln Leu Leu
                85              90              95


Ser Pro Ser Gly Ser Lys Ile Asn Thr Gly Ala Asp Gly Leu Gln Arg
            100             105             110


Leu Asp His Val Val Lys Ser Ala Glu Lys Arg Gly Val Lys Leu Ile
        115             120             125


Ile Asn Phe Val Asn Asn Trp Asp Asp Tyr Gly Gly Met Asn Ala Tyr
    130             135             140


Val Lys Ala Phe Gly Gly Thr Lys Glu Gly Trp Tyr Thr Asn Ala Lys
145             150             155             160


Ala Gln Gln Gln Tyr Lys Lys Tyr Ile Lys Ala Val Val Ser Arg Tyr
                165             170             175


Ala Lys Ser Pro Ala Val Phe Ala Trp Glu Leu Ala Asn Glu Pro Arg
            180             185             190


Cys Lys Gly Cys Ser Thr Asp Val Ile Tyr Lys Trp Ala Thr Glu Ile
        195             200             205


Ser Ala Tyr Ile Arg Lys Leu Asp Pro Ser His Met Ile Thr Leu Gly
    210             215             220


Asp Glu Gly Phe Gly Leu Pro Gly Asp Thr Thr Tyr Pro Tyr Ser Tyr
225             230             235             240


Thr Glu Gly Val Asp Phe Val Lys Asn Leu Gly Ile Lys Asn Leu Asp
                245             250             255


Phe Gly Thr Phe His Met Tyr Pro Asp Ser Trp Gly Val Pro Tyr Ser
            260             265             270


Phe Gly Glu Gly Trp Ile Lys Asn His Ala Ala Ala Cys Lys Pro Ala
```

275                    280                      285

Gly Lys Pro Cys Leu Leu Glu Glu Tyr Gly Ala Glu His Ser Cys Asp
    290                  295                  300

Ile Gln Lys Pro Trp Gln Gln Ala Ser Leu Ala Leu Ala Lys Glu Gly
305                  310                  315                  320

Met Ser Gly Asp Leu Phe Trp Gln Trp Gly Asp Ala Leu Ser Phe Gly
                325                  330                  335

Gln Ser Pro Asn Asp Gly His Thr Val Tyr Tyr Gly Ser Glu Leu Ala
            340                  345                  350

Gln Cys Leu Val Thr Asp His Val Lys Glu Ile Asn Ala Ser Ser
        355                  360                  365

<210> 8
<211> 350
<212> PRT
<213> Chaetomium virescens

<400> 8

```
Leu Pro Ser Ala Lys Ala Ala Ser Ala Thr Thr Asn Gly Thr Arg Phe
1               5               10              15

Thr Val Asp Gly Lys Thr Gly Tyr Phe Ala Gly Thr Asn Ser Tyr Trp
        20              25              30

Ile Gly Phe Leu Thr Asn Asn Lys Asp Ile Asp Thr Thr Leu Asp His
        35              40              45

Ile Ser Ser Ser Gly Leu Lys Ile Leu Arg Val Trp Gly Phe Asn Asp
    50              55              60

Val Asn Thr Lys Pro Ser Asp Gly Thr Val Trp Tyr Gln Leu Leu Ser
65              70              75              80

Pro Ser Gly Ser Lys Ile Asn Thr Gly Ala Asp Gly Leu Gln Arg Leu
            85              90              95

Asp His Val Val Lys Ser Ala Glu Lys Arg Gly Val Lys Leu Ile Ile
        100             105             110

Asn Phe Val Asn Asn Trp Asp Asp Tyr Gly Gly Met Asn Ala Tyr Val
        115             120             125

Lys Ala Phe Gly Gly Thr Lys Glu Gly Trp Tyr Thr Asn Ala Lys Ala
```

<pre>
            130                     135                       140


        Gln Gln Gln Tyr Lys Lys Tyr Ile Lys Ala Val Val Ser Arg Tyr Ala
        145                 150                 155                 160


        Lys Ser Pro Ala Val Phe Ala Trp Glu Leu Ala Asn Glu Pro Arg Cys
                        165                 170                 175


        Lys Gly Cys Ser Thr Asp Val Ile Tyr Lys Trp Ala Thr Glu Ile Ser
                    180                 185                 190


        Ala Tyr Ile Arg Lys Leu Asp Pro Ser His Met Ile Thr Leu Gly Asp
                195                 200                 205


        Glu Gly Phe Gly Leu Pro Gly Asp Thr Thr Tyr Pro Tyr Ser Tyr Thr
            210                 215                 220


        Glu Gly Val Asp Phe Val Lys Asn Leu Gly Ile Lys Asn Leu Asp Phe
        225                 230                 235                 240


        Gly Thr Phe His Met Tyr Pro Asp Ser Trp Gly Val Pro Tyr Ser Phe
                        245                 250                 255


        Gly Glu Gly Trp Ile Lys Asn His Ala Ala Ala Cys Lys Pro Ala Gly
                    260                 265                 270


        Lys Pro Cys Leu Leu Glu Glu Tyr Gly Ala Glu His Ser Cys Asp Ile
                    275                 280                 285


        Gln Lys Pro Trp Gln Gln Ala Ser Leu Ala Leu Ala Lys Glu Gly Met
            290                 295                 300


        Ser Gly Asp Leu Phe Trp Gln Trp Gly Asp Ala Leu Ser Phe Gly Gln
        305                 310                 315                 320


        Ser Pro Asn Asp Gly His Thr Val Tyr Tyr Gly Ser Glu Leu Ala Gln
                        325                 330                 335


        Cys Leu Val Thr Asp His Val Lys Glu Ile Asn Ala Ser Ser
                    340                 345                 350
</pre>

<210> 9
<211> 40
<212> DNA
<213> Artificial Sequence

<220>

<223> Primer

<400> 9
acacaactgg ggatccacca tgaaggcaat cctcacagcc 40

<210> 10
<211> 37
<212> DNA
<213> Artificial Sequence

<220>
<223> Primer

<400> 10
ccctctagat ctcgagtgcg tatcacggga cttcaga 37

<210> 11
<211> 1203
<212> DNA
<213> Sordaria macrospora

<220>
<221> CDS
<222> (1)..(781)

<220>
<221> Intron
<222> (782)..(838)

<220>
<221> CDS
<222> (839)..(928)

<220>
<221> Intron
<222> (929)..(988)

<220>
<221> CDS
<222> (989)..(1200)

<400> 11

```
atg aag tcc ttg ttc acc ctc gcc ctc ggc ttg cta tca ttg gtc tca        48
Met Lys Ser Leu Phe Thr Leu Ala Leu Gly Leu Leu Ser Leu Val Ser
1               5               10              15

gct gcg cct ccc act gtc aat ggc aca cgc ttc tcc atc gac ggc aaa        96
Ala Ala Pro Pro Thr Val Asn Gly Thr Arg Phe Ser Ile Asp Gly Lys
            20              25              30

acg ggg tac ttt gcc ggt acc aac tcg tac tgg atc ggc ttc cta acc       144
Thr Gly Tyr Phe Ala Gly Thr Asn Ser Tyr Trp Ile Gly Phe Leu Thr
        35              40              45

aaa aac cga gat gtc gac acc gtc ctt gac cac atc tcc tcc tcc gga       192
Lys Asn Arg Asp Val Asp Thr Val Leu Asp His Ile Ser Ser Ser Gly
        50              55              60

ctc aaa atc ctg cgc atc tgg ggc ttc aac gac gtc acc cgc aag cca       240
Leu Lys Ile Leu Arg Ile Trp Gly Phe Asn Asp Val Thr Arg Lys Pro
65              70              75              80
```

```
gcc tcc ggc acc gtg tgg tac cag ctc ctc tcc tcg tcc ggt tcc cag        288
Ala Ser Gly Thr Val Trp Tyr Gln Leu Leu Ser Ser Ser Gly Ser Gln
                85              90                  95

atc aac acc ggt gcc gat ggc ctg cag cgc ctg gac tac gtc gtc cag        336
Ile Asn Thr Gly Ala Asp Gly Leu Gln Arg Leu Asp Tyr Val Val Gln
            100             105             110

tcc gcc gaa aag cgt ggt gtc aag ctc atc att aac ttt gtc aac aac        384
Ser Ala Glu Lys Arg Gly Val Lys Leu Ile Ile Asn Phe Val Asn Asn
        115             120             125

tgg agc gac tac ggc ggc atg cca gcc tac gtg act gca ttc gga ggt        432
Trp Ser Asp Tyr Gly Gly Met Pro Ala Tyr Val Thr Ala Phe Gly Gly
    130             135             140

tcc cag gag agc tgg tac acc aac agt cgg gcg cag gcg cag tac aag        480
Ser Gln Glu Ser Trp Tyr Thr Asn Ser Arg Ala Gln Ala Gln Tyr Lys
145             150             155             160

gcc tac att gcc gct gtt gtc aac cgc tat atc aac tct tcc gct gtc        528
Ala Tyr Ile Ala Ala Val Val Asn Arg Tyr Ile Asn Ser Ser Ala Val
            165             170             175

ttt gcc tgg gag ctg gcg aac gag ccc cgc tgc aag gga tgt agc act        576
Phe Ala Trp Glu Leu Ala Asn Glu Pro Arg Cys Lys Gly Cys Ser Thr
            180             185             190

gat gtt att tac aag tgg gca act gac atc tca gct tac atc cgc agt        624
Asp Val Ile Tyr Lys Trp Ala Thr Asp Ile Ser Ala Tyr Ile Arg Ser
            195             200             205

ttg gat tgc aac cac atg atc acc ctc gga gac gaa ggg ttt gga ctt        672
Leu Asp Cys Asn His Met Ile Thr Leu Gly Asp Glu Gly Phe Gly Leu
    210             215             220

ccc ggg gcg acc agc tat ccg tat caa acc agc gaa ggc gtg gat ttt        720
Pro Gly Ala Thr Ser Tyr Pro Tyr Gln Thr Ser Glu Gly Val Asp Phe
225             230             235             240

gtc aag aat ctg gcc att aag aac ttg gat ttt ggc act ttc cac ttc        768
Val Lys Asn Leu Ala Ile Lys Asn Leu Asp Phe Gly Thr Phe His Phe
            245             250             255

tat ccg caa agc t gtacgtaaca tgaaactccc atgtcaggga atcaacactg          821
Tyr Pro Gln Ser
            260

accgtgacta ttcttag gg  ggg gtg ggc aat gct gtt ggt gca gct tgg        870
                    Trp Gly Val Gly Asn Ala Val Gly Ala Ala Trp
                        265             270

atc aaa gac cat gcc tcg gct tgc aag aag gcc ggg aag cct tgt cta        918
Ile Lys Asp His Ala Ser Ala Cys Lys Lys Ala Gly Lys Pro Cys Leu
            275             280             285

ttt gag gag t gtaagtggat gctgtgagcc agtctgattt gcaggtagct            968
Phe Glu Glu
            290

aacgaatgct attcttccag at  ggc acc tca acc gat cac tgc acc atc gag    1020
                        Tyr Gly Thr Ser Thr Asp His Cys Thr Ile Glu
                            295             300
```

53

```
cga cct tgg caa caa gcc tcc ctc caa gct gcc acg gag ggc atg gca        1068
Arg Pro Trp Gln Gln Ala Ser Leu Gln Ala Ala Thr Glu Gly Met Ala
            305             310             315

gct gac ttg ttt tgg caa tgg gga gat aat ctg agc acg ggg cag aca        1116
Ala Asp Leu Phe Trp Gln Trp Gly Asp Asn Leu Ser Thr Gly Gln Thr
            320             325             330

cac aat gac ggg aac acg atc tat tat gga tca gcc gat gcc act tgc        1164
His Asn Asp Gly Asn Thr Ile Tyr Tyr Gly Ser Ala Asp Ala Thr Cys
            335             340             345

ttg att acc gag cat gtc agg gcc atc aac tcc ctc tag                    1203
Leu Ile Thr Glu His Val Arg Ala Ile Asn Ser Leu
350             355             360
```

<210> 12
<211> 361
<212> PRT
<213> Sordaria macrospora

<400> 12

```
Met Lys Ser Leu Phe Thr Leu Ala Leu Gly Leu Leu Ser Leu Val Ser
1               5                   10                  15

Ala Ala Pro Pro Thr Val Asn Gly Thr Arg Phe Ser Ile Asp Gly Lys
            20                  25                  30

Thr Gly Tyr Phe Ala Gly Thr Asn Ser Tyr Trp Ile Gly Phe Leu Thr
        35                  40                  45

Lys Asn Arg Asp Val Asp Thr Val Leu Asp His Ile Ser Ser Ser Gly
    50                  55                  60

Leu Lys Ile Leu Arg Ile Trp Gly Phe Asn Asp Val Thr Arg Lys Pro
65                  70                  75                  80

Ala Ser Gly Thr Val Trp Tyr Gln Leu Leu Ser Ser Ser Gly Ser Gln
            85                  90                  95

Ile Asn Thr Gly Ala Asp Gly Leu Gln Arg Leu Asp Tyr Val Val Gln
            100                 105                 110

Ser Ala Glu Lys Arg Gly Val Lys Leu Ile Ile Asn Phe Val Asn Asn
        115                 120                 125

Trp Ser Asp Tyr Gly Gly Met Pro Ala Tyr Val Thr Ala Phe Gly Gly
    130                 135                 140

Ser Gln Glu Ser Trp Tyr Thr Asn Ser Arg Ala Gln Ala Gln Tyr Lys
145                 150                 155                 160
```

```
Ala Tyr Ile Ala Ala Val Val Asn Arg Tyr Ile Asn Ser Ser Ala Val
                165             170             175

Phe Ala Trp Glu Leu Ala Asn Glu Pro Arg Cys Lys Gly Cys Ser Thr
            180             185             190

Asp Val Ile Tyr Lys Trp Ala Thr Asp Ile Ser Ala Tyr Ile Arg Ser
            195             200             205

Leu Asp Cys Asn His Met Ile Thr Leu Gly Asp Glu Gly Phe Gly Leu
    210             215             220

Pro Gly Ala Thr Ser Tyr Pro Tyr Gln Thr Ser Glu Gly Val Asp Phe
225             230             235             240

Val Lys Asn Leu Ala Ile Lys Asn Leu Asp Phe Gly Thr Phe His Phe
            245             250             255

Tyr Pro Gln Ser Trp Gly Val Gly Asn Ala Val Gly Ala Ala Trp Ile
            260             265             270

Lys Asp His Ala Ser Ala Cys Lys Lys Ala Gly Lys Pro Cys Leu Phe
            275             280             285

Glu Glu Tyr Gly Thr Ser Thr Asp His Cys Thr Ile Glu Arg Pro Trp
            290             295             300

Gln Gln Ala Ser Leu Gln Ala Ala Thr Glu Gly Met Ala Ala Asp Leu
305             310             315             320

Phe Trp Gln Trp Gly Asp Asn Leu Ser Thr Gly Gln Thr His Asn Asp
            325             330             335

Gly Asn Thr Ile Tyr Tyr Gly Ser Ala Asp Ala Thr Cys Leu Ile Thr
            340             345             350

Glu His Val Arg Ala Ile Asn Ser Leu
            355             360
```

<210> 13
<211> 344
<212> PRT
<213> Sordaria macrospora

<400> 13

```
Ala Pro Pro Thr Val Asn Gly Thr Arg Phe Ser Ile Asp Gly Lys Thr
1               5               10              15
```

```
Gly Tyr Phe Ala Gly Thr Asn Ser Tyr Trp Ile Gly Phe Leu Thr Lys
          20                  25                  30

Asn Arg Asp Val Asp Thr Val Leu Asp His Ile Ser Ser Ser Gly Leu
          35                  40                  45

Lys Ile Leu Arg Ile Trp Gly Phe Asn Asp Val Thr Arg Lys Pro Ala
          50                  55                  60

Ser Gly Thr Val Trp Tyr Gln Leu Leu Ser Ser Ser Gly Ser Gln Ile
65                  70                  75                  80

Asn Thr Gly Ala Asp Gly Leu Gln Arg Leu Asp Tyr Val Val Gln Ser
              85                  90                  95

Ala Glu Lys Arg Gly Val Lys Leu Ile Ile Asn Phe Val Asn Asn Trp
          100                 105                 110

Ser Asp Tyr Gly Gly Met Pro Ala Tyr Val Thr Ala Phe Gly Gly Ser
          115                 120                 125

Gln Glu Ser Trp Tyr Thr Asn Ser Arg Ala Gln Ala Gln Tyr Lys Ala
          130                 135                 140

Tyr Ile Ala Ala Val Val Asn Arg Tyr Ile Asn Ser Ser Ala Val Phe
145                 150                 155                 160

Ala Trp Glu Leu Ala Asn Glu Pro Arg Cys Lys Gly Cys Ser Thr Asp
              165                 170                 175

Val Ile Tyr Lys Trp Ala Thr Asp Ile Ser Ala Tyr Ile Arg Ser Leu
          180                 185                 190

Asp Cys Asn His Met Ile Thr Leu Gly Asp Glu Gly Phe Gly Leu Pro
          195                 200                 205

Gly Ala Thr Ser Tyr Pro Tyr Gln Thr Ser Glu Gly Val Asp Phe Val
          210                 215                 220

Lys Asn Leu Ala Ile Lys Asn Leu Asp Phe Gly Thr Phe His Phe Tyr
225                 230                 235                 240

Pro Gln Ser Trp Gly Val Gly Asn Ala Val Gly Ala Ala Trp Ile Lys
              245                 250                 255

Asp His Ala Ser Ala Cys Lys Lys Ala Gly Lys Pro Cys Leu Phe Glu
```

                    260                    265                    270

        Glu Tyr Gly Thr Ser Thr Asp His Cys Thr Ile Glu Arg Pro Trp Gln
                275                280                285

        Gln Ala Ser Leu Gln Ala Ala Thr Glu Gly Met Ala Ala Asp Leu Phe
            290                295                300

        Trp Gln Trp Gly Asp Asn Leu Ser Thr Gly Gln Thr His Asn Asp Gly
        305                310                315                320

        Asn Thr Ile Tyr Tyr Gly Ser Ala Asp Ala Thr Cys Leu Ile Thr Glu
                325                330                335

        His Val Arg Ala Ile Asn Ser Leu
                340

<210> 14
<211> 43
<212> DNA
<213> Artificial Sequence

<220>
<223> Primer

<400> 14
acacaactgg ggatccacca tgaagtcctt gttcaccctc gcc 43

<210> 15
<211> 35
<212> DNA
<213> Artificial Sequence

<220>
<223> Primer

<400> 15
ccctctagat ctcgaggtac gcagccacgg cgaca 35

<210> 16
<211> 309
<212> PRT
<213> Bacillus sp.

<400> 16

Ala Asn Ser Gly Phe Tyr Val Ser Gly Thr Thr Leu Tyr Asp Ala Asn
1               5                   10                  15

Gly Asn Pro Phe Val Met Arg Gly Ile Asn His Gly His Ala Trp Tyr
            20                  25                  30

Lys Asp Gln Ala Thr Thr Ala Ile Glu Gly Ile Ala Asn Thr Gly Ala

35 40 45

Asn Thr Val Arg Ile Val Leu Ser Asp Gly Gly Gln Trp Thr Lys Asp
50 55 60

Asp Ile His Thr Val Arg Asn Leu Ile Ser Leu Ala Glu Asp Asn His
65 70 75 80

Leu Val Ala Val Leu Glu Val His Asp Ala Thr Gly Tyr Asp Ser Ile
85 90 95

Ala Ser Leu Asn Arg Ala Val Asp Tyr Trp Ile Glu Met Arg Ser Ala
100 105 110

Leu Ile Gly Lys Glu Asp Thr Val Ile Ile Asn Ile Ala Asn Glu Trp
115 120 125

Phe Gly Ser Trp Glu Gly Asp Ala Trp Ala Asp Gly Tyr Lys Gln Ala
130 135 140

Ile Pro Arg Leu Arg Asn Ala Gly Leu Asn His Thr Leu Met Val Asp
145 150 155 160

Ala Ala Gly Trp Gly Gln Phe Pro Gln Ser Ile His Asp Tyr Gly Arg
165 170 175

Glu Val Phe Asn Ala Asp Pro Gln Arg Asn Thr Met Phe Ser Ile His
180 185 190

Met Tyr Glu Tyr Ala Gly Gly Asn Ala Ser Gln Val Arg Thr Asn Ile
195 200 205

Asp Arg Val Leu Asn Gln Asp Leu Ala Leu Val Ile Gly Glu Phe Gly
210 215 220

His Arg His Thr Asn Gly Asp Val Asp Glu Ala Thr Ile Met Ser Tyr
225 230 235 240

Ser Glu Gln Arg Gly Val Gly Trp Leu Ala Trp Ser Trp Lys Gly Asn
245 250 255

Gly Pro Glu Trp Glu Tyr Leu Asp Leu Ser Asn Asp Trp Ala Gly Asn
260 265 270

Asn Leu Thr Ala Trp Gly Asn Thr Ile Val Asn Gly Pro Tyr Gly Leu
275 280 285

```
Arg Glu Thr Ser Arg Leu Ser Thr Val Phe Thr Gly Gly Gly Ser Asp
    290                 295             300

Gly Gly Thr Ser Pro
305
```

<210> 17
<211> 335
<212> PRT
<213> Caldicellulosiruptor saccharolyticus

<400> 17

```
Met Lys Lys Pro Leu Gly Lys Ile Val Ala Ser Thr Ala Leu Leu Ile
1               5               10                  15

Ser Val Ala Phe Ser Ser Ser Ile Ala Ser Ala Ala Thr Ser Asn Asp
            20              25                  30

Gly Val Val Lys Ile Asp Thr Ser Thr Leu Ile Gly Thr Asn His Ala
        35              40                  45

His Cys Trp Tyr Arg Asp Arg Leu Asp Thr Ala Leu Arg Gly Ile Arg
    50              55                  60

Ser Trp Gly Met Asn Ser Val Arg Val Val Leu Ser Asn Gly Tyr Arg
65              70                  75                  80

Trp Thr Lys Ile Pro Ala Ser Glu Val Ala Asn Ile Ile Ser Leu Ser
                85              90                  95

Arg Ser Leu Gly Phe Lys Ala Ile Ile Leu Glu Val His Asp Thr Thr
            100             105                 110

Gly Tyr Gly Glu Asp Gly Ala Ala Cys Ser Leu Ala Gln Ala Val Glu
            115             120                 125

Tyr Trp Lys Glu Ile Lys Ser Val Leu Asp Gly Asn Glu Asp Phe Val
    130             135                 140

Ile Ile Asn Ile Gly Asn Glu Pro Tyr Gly Asn Asn Tyr Gln Asn
145                 150                 155             160

Trp Val Asn Asp Thr Lys Asn Ala Ile Lys Ala Leu Arg Asp Ala Gly
                165                 170                 175

Phe Lys His Thr Ile Met Val Asp Ala Pro Asn Trp Gly Gln Asp Trp
            180                 185                 190
```

```
Ser Asn Thr Met Arg Asp Asn Ala Gln Ser Ile Met Glu Ala Asp Pro
        195             200             205

Leu Arg Asn Leu Val Phe Ser Ile His Met Tyr Gly Val Tyr Asn Thr
        210             215             220

Ala Ser Lys Val Glu Glu Tyr Ile Lys Ser Phe Val Asp Lys Gly Leu
225             230             235             240

Pro Leu Val Ile Gly Glu Phe Gly His Gln His Thr Asp Gly Asp Pro
                245             250             255

Asp Glu Glu Ala Ile Val Arg Tyr Ala Lys Gln Tyr Lys Ile Gly Leu
            260             265             270

Phe Ser Trp Ser Trp Cys Gly Asn Ser Ser Tyr Val Gly Tyr Leu Asp
        275             280             285

Met Val Asn Asn Trp Asp Pro Asn Asn Pro Thr Pro Trp Gly Gln Trp
    290             295             300

Tyr Lys Thr Asn Ala Ile Gly Thr Ser Ser Thr Pro Thr Pro Thr Ser
305             310             315             320

Thr Val Thr Pro Thr Pro Pro Pro Arg Gln His Gln His Arg Gln
            325             330             335
```

<210> 18
<211> 292
<212> PRT
<213> Caldicellulosiruptor saccharolyticus

<400> 18

```
Ala Thr Ser Asn Asp Gly Val Val Lys Ile Asp Thr Ser Thr Leu Ile
1               5               10              15

Gly Thr Asn His Ala His Cys Trp Tyr Arg Asp Arg Leu Asp Thr Ala
            20              25              30

Leu Arg Gly Ile Arg Ser Trp Gly Met Asn Ser Val Arg Val Val Leu
        35              40              45

Ser Asn Gly Tyr Arg Trp Thr Lys Ile Pro Ala Ser Glu Val Ala Asn
    50              55              60

Ile Ile Ser Leu Ser Arg Ser Leu Gly Phe Lys Ala Ile Ile Leu Glu
65              70              75              80
```

```
Val His Asp Thr Thr Gly Tyr Gly Glu Asp Gly Ala Ala Cys Ser Leu
            85                90                95

Ala Gln Ala Val Glu Tyr Trp Lys Glu Ile Lys Ser Val Leu Asp Gly
            100               105               110

Asn Glu Asp Phe Val Ile Ile Asn Ile Gly Asn Glu Pro Tyr Gly Asn
            115               120               125

Asn Asn Tyr Gln Asn Trp Val Asn Asp Thr Lys Asn Ala Ile Lys Ala
    130               135               140

Leu Arg Asp Ala Gly Phe Lys His Thr Ile Met Val Asp Ala Pro Asn
145               150               155               160

Trp Gly Gln Asp Trp Ser Asn Thr Met Arg Asp Asn Ala Gln Ser Ile
            165               170               175

Met Glu Ala Asp Pro Leu Arg Asn Leu Val Phe Ser Ile His Met Tyr
            180               185               190

Gly Val Tyr Asn Thr Ala Ser Lys Val Glu Glu Tyr Ile Lys Ser Phe
            195               200               205

Val Asp Lys Gly Leu Pro Leu Val Ile Gly Glu Phe Gly His Gln His
    210               215               220

Thr Asp Gly Asp Pro Asp Glu Glu Ala Ile Val Arg Tyr Ala Lys Gln
225               230               235               240

Tyr Lys Ile Gly Leu Phe Ser Trp Ser Trp Cys Gly Asn Ser Ser Tyr
            245               250               255

Val Gly Tyr Leu Asp Met Val Asn Asn Trp Asp Pro Asn Asn Pro Thr
            260               265               270

Pro Trp Gly Gln Trp Tyr Lys Thr Asn Ala Ile Gly Thr Ser Ser Thr
            275               280               285

Pro Thr Pro Thr
    290
```

<210> 19
<211> 418
<212> PRT
<213> Trichoderma reesei

<400> 19

```
Ala Val Leu Gln Pro Val Pro Arg Ala Ser Ser Phe Val Thr Ile Ser
1               5               10                  15

Gly Thr Gln Phe Asn Ile Asp Gly Lys Val Gly Tyr Phe Ala Gly Thr
            20              25              30

Asn Cys Tyr Trp Cys Ser Phe Leu Thr Asn His Ala Asp Val Asp Ser
        35              40              45

Thr Phe Ser His Ile Ser Ser Ser Gly Leu Lys Val Val Arg Val Trp
    50              55              60

Gly Phe Asn Asp Val Asn Thr Gln Pro Ser Pro Gly Gln Ile Trp Phe
65              70              75              80

Gln Lys Leu Ser Ala Thr Gly Ser Thr Ile Asn Thr Gly Ala Asp Gly
            85              90              95

Leu Gln Thr Leu Asp Tyr Val Val Gln Ser Ala Glu Gln His Asn Leu
            100             105             110

Lys Leu Ile Ile Pro Phe Val Asn Asn Trp Ser Asp Tyr Gly Gly Ile
        115             120             125

Asn Ala Tyr Val Asn Ala Phe Gly Gly Asn Ala Thr Thr Trp Tyr Thr
    130             135             140

Asn Thr Ala Ala Gln Thr Gln Tyr Arg Lys Tyr Val Gln Ala Val Val
145             150             155             160

Ser Arg Tyr Ala Asn Ser Thr Ala Ile Phe Ala Trp Glu Leu Gly Asn
            165             170             175

Glu Pro Arg Cys Asn Gly Cys Ser Thr Asp Val Ile Val Gln Trp Ala
            180             185             190

Thr Ser Val Ser Gln Tyr Val Lys Ser Leu Asp Ser Asn His Leu Val
    195             200             205

Thr Leu Gly Asp Glu Gly Leu Gly Leu Ser Thr Gly Asp Gly Ala Tyr
    210             215             220

Pro Tyr Thr Tyr Gly Glu Gly Thr Asp Phe Ala Lys Asn Val Gln Ile
225             230             235             240

Lys Ser Leu Asp Phe Gly Thr Phe His Leu Tyr Pro Asp Ser Trp Gly
            245             250             255
```

```
        Thr Asn Tyr Thr Trp Gly Asn Gly Trp Ile Gln Thr His Ala Ala Ala
                    260             265             270


        Cys Leu Ala Ala Gly Lys Pro Cys Val Phe Glu Glu Tyr Gly Ala Gln
                    275             280             285


        Gln Asn Pro Cys Thr Asn Glu Ala Pro Trp Gln Thr Thr Ser Leu Thr
                    290             295             300


        Thr Arg Gly Met Gly Gly Asp Met Phe Trp Gln Trp Gly Asp Thr Phe
            305             310             315             320


        Ala Asn Gly Ala Gln Ser Asn Ser Asp Pro Tyr Thr Val Trp Tyr Asn
                    325             330             335


        Ser Ser Asn Trp Gln Cys Leu Val Lys Asn His Val Asp Ala Ile Asn
                    340             345             350


        Gly Gly Thr Thr Thr Pro Pro Pro Val Ser Ser Thr Thr Thr Thr Ser
                    355             360             365


        Ser Arg Thr Ser Ser Thr Pro Pro Pro Pro Gly Gly Ser Cys Ser Pro
            370             375             380


        Leu Tyr Gly Gln Cys Gly Gly Ser Gly Tyr Thr Gly Pro Thr Cys Cys
        385             390             395             400


        Ala Gln Gly Thr Cys Ile Tyr Ser Asn Tyr Trp Tyr Ser Gln Cys Leu
                    405             410             415


        Asn Thr
```

<210> 20
<211> 355
<212> PRT
<213> Trichoderma reesei

<400> 20

```
        Ala Val Leu Gln Pro Val Pro Arg Ala Ser Ser Phe Val Thr Ile Ser
        1               5               10              15


        Gly Thr Gln Phe Asn Ile Asp Gly Lys Val Gly Tyr Phe Ala Gly Thr
                    20              25              30


        Asn Cys Tyr Trp Cys Ser Phe Leu Thr Asn His Ala Asp Val Asp Ser
                    35              40              45
```

Thr Phe Ser His Ile Ser Ser Ser Gly Leu Lys Val Val Arg Val Trp
50          55              60

Gly Phe Asn Asp Val Asn Thr Gln Pro Ser Pro Gly Gln Ile Trp Phe
65          70              75                      80

Gln Lys Leu Ser Ala Thr Gly Ser Thr Ile Asn Thr Gly Ala Asp Gly
85              90                  95

Leu Gln Thr Leu Asp Tyr Val Val Gln Ser Ala Glu Gln His Asn Leu
100             105                 110

Lys Leu Ile Ile Pro Phe Val Asn Asn Trp Ser Asp Tyr Gly Gly Ile
115             120                 125

Asn Ala Tyr Val Asn Ala Phe Gly Gly Asn Ala Thr Thr Trp Tyr Thr
130             135                 140

Asn Thr Ala Ala Gln Thr Gln Tyr Arg Lys Tyr Val Gln Ala Val Val
145             150                 155                 160

Ser Arg Tyr Ala Asn Ser Thr Ala Ile Phe Ala Trp Glu Leu Gly Asn
165             170                 175

Glu Pro Arg Cys Asn Gly Cys Ser Thr Asp Val Ile Val Gln Trp Ala
180             185                 190

Thr Ser Val Ser Gln Tyr Val Lys Ser Leu Asp Ser Asn His Leu Val
195             200                 205

Thr Leu Gly Asp Glu Gly Leu Gly Leu Ser Thr Gly Asp Gly Ala Tyr
210             215                 220

Pro Tyr Thr Tyr Gly Glu Gly Thr Asp Phe Ala Lys Asn Val Gln Ile
225             230                 235                 240

Lys Ser Leu Asp Phe Gly Thr Phe His Leu Tyr Pro Asp Ser Trp Gly
245             250                 255

Thr Asn Tyr Thr Trp Gly Asn Gly Trp Ile Gln Thr His Ala Ala Ala
260             265                 270

Cys Leu Ala Ala Gly Lys Pro Cys Val Phe Glu Glu Tyr Gly Ala Gln
275             280                 285

Gln Asn Pro Cys Thr Asn Glu Ala Pro Trp Gln Thr Thr Ser Leu Thr
290             295                 300

```
        Thr Arg Gly Met Gly Gly Asp Met Phe Trp Gln Trp Gly Asp Thr Phe
        305             310             315                     320


        Ala Asn Gly Ala Gln Ser Asn Ser Asp Pro Tyr Thr Val Trp Tyr Asn
                        325             330                     335


        Ser Ser Asn Trp Gln Cys Leu Val Lys Asn His Val Asp Ala Ile Asn
                        340             345             350


        Gly Gly Thr
                355
```